# EUROPEAN PATENT SPECIFICATION

(11) **EP 4 516 200 B1**
(45) Date of publication and mention of the grant of the patent: **29.04.2026**
(21) Application number: 24197240.5
(22) Date of filing: 29.08.2024
(51) Int. Cl.: A61B 1/00, A61B 1/01, A61B 1/015

(54) **OVERTUBE**
ÜBERROHR
SURTUBE

(30) Priority: 29.08.2023 JP 2023139259
(43) Date of publication of application: 05.03.2025
(73) Proprietor: FUJIFILM Corporation, Tokyo 106-8620 (JP)
(72) Inventor: ABE, Shinya, c/o FUJIFILM Corporation, 798, Miyanodai, Kaisei-machi, Ashigarakami-gun, Kanagawa, 258-8538 (JP); FURUTA, Ayana, c/o FUJIFILM Corporation, 798, Miyanodai, Kaisei-machi, Ashigarakami-gun, Kanagawa, 258-8538 (JP)
(74) Representative: HGF

(56) References cited:
- WO-A1-2012/043034
- WO-A1-2022/181200
- US-A1- 2012 184 817

## Description

### BACKGROUND OF THE INVENTION

### 1. Field of the Invention

The present disclosure relates to an overtube having an insertion path into which a medical instrument can be inserted.

### 2. Description of the Related Art

An overtube having an insertion path into which a medical instrument such as an insertion part of an endoscope (hereinafter, also referred to as an "endoscope insertion part") can be inserted is known.

For example, WO2022/181200A discloses a variable stiffness overtube (guide tube). The overtube has a tube body in which an outer tube covers a periphery of an inner tube, and a shape-deformable body that can be deformed following a shape of the tube body is provided between the outer tube and the inner tube.

In the overtube disclosed in WO2022/181200A, in a case where an inner space between the outer tube and the inner tube is depressurized, the overtube is in a rigid state (stiffened state) in a state in which a shape of the shape-deformable body is maintained. Meanwhile, in a case where the depressurization of the inner space between the outer tube and the inner tube is released, the shape retention by the shape-deformable body is released, and the overtube is in a soft state (softened state). Accordingly, the endoscope insertion part can be guided into the body by performing a movement operation of moving the overtube and the endoscope insertion part forward and backward relative to each other while switching the overtube between the stiffened state and the softened state in a state in which the endoscope insertion part is inserted into the overtube. US 2012/184817 discloses an insertion aid including a long guide portion arranged relatively movable in a longitudinal direction along an insertion portion of an endoscope to be inserted into a subject, the guide portion being able to undergo a state change between a first state where flexural rigidity is low, and a second state where flexural rigidity is high, wherein the guide portion includes a strength variable member arranged to extend along a longitudinal direction of the guide portion and whose strength varies according to temperature, and wherein a flexural rigidity of a proximal end side of the guide portion is set to be relatively higher than the flexural rigidity of a distal end side by setting a boundary temperature at which a proximal end side of the strength variable member starts hardening to be relatively lower than the boundary temperature of a distal end side of the strength variable member.

### SUMMARY OF THE INVENTION

However, in the variable stiffness overtube as disclosed in WO2022/181200A, the entire overtube becomes stiff in a longitudinal direction in a case where the overtube is switched from the softened state to the stiffened state. Therefore, insertability of a distal end side of the overtube into a digestive tract (particularly an upper digestive tract) deteriorates. In addition, the same applies to a case where the overtube is in the softened state. Therefore, the movement operation may be complicated and difficult, and the endoscope insertion part may not be able to be guided in a desired direction.

The present invention has been made in view of such circumstances, and an object thereof is to provide an overtube with improved insertability of a distal end side of the overtube into a digestive tract.

The present disclosure includes the following inventions.

According to a first aspect, there is provided an overtube having an insertion path into which a medical instrument is inserted, the overtube comprising: a supply and discharge port that is located on a proximal end side and through which a fluid is supplied and discharged; a first region that is located on a distal end side with respect to the supply and discharge port and includes an outer tube having a flexibility, an inner tube having a flexibility, and a shape-deformable body that is deformable and is provided between the outer tube and the inner tube; and a second region that is located on the distal end side with respect to the first region and includes the outer tube and the inner tube, in which the first region is switchable between a first state in which the fluid is supplied to a space between the outer tube and the inner tube via the supply and discharge port and a second state that is stiffer than the first state and in which the fluid is discharged, and the second region is softer than the second state of the first region.

According to a second aspect, in the overtube of the first aspect, the second region is softer than the first state of the first region.

According to a third aspect, in the overtube of the first or the second aspect, a distal end side of the second region is softer than a proximal end side of the second region.

According to a fourth aspect, in the overtube of any one of the first to third aspects, the second region includes the shape-deformable body, and the second region is switchable between the first state in which the fluid is supplied to the space between the outer tube and the inner tube via the supply and discharge port and the second state in which the fluid is discharged.

According to a fifth aspect, in the overtube of any one of the first to fourth aspects, the first state of the second region is softer than the first state of the first region, and the second state of the second region is softer than the second state of the first region.

According to a sixth aspect, in the overtube of any one of the first to fifth aspects, the first region has an interlayer that is provided between the outer tube and the inner tube and is in contact with the shape-deformable body, and a first contact surface provided in the shape-deformable body and a second contact surface provided in the interlayer and facing the first contact surface come into contact with each other in the second state, so that the first region becomes stiff.

According to a seventh aspect, in the overtube of any one of the first to sixth aspects, the second region includes the interlayer different in configuration from the interlayer of the first region.

According to an eighth aspect, in the overtube of the seventh aspect, the interlayer of the second region has a constant flexibility along a longitudinal direction.

According to a ninth aspect, in the overtube of the seventh aspect, the interlayer of the second region is softer on a distal end side than on a proximal end side.

According to a tenth aspect, in the overtube of the ninth aspect, the interlayer of the second region includes a first interlayer provided on the distal end side and a second interlayer provided on the proximal end side, and the first interlayer is softer than the second interlayer.

According to an eleventh aspect, in the overtube of the ninth aspect, the interlayer of the second region is gradually softer toward the distal end side.

According to a twelfth aspect, in the overtube of any one of the seventh to eleventh aspects, the interlayer is formed of a sheet material provided from the first region to the second region, and a cut portion is provided in the sheet material of the second region.

According to a thirteenth aspect, in the overtube of the twelfth aspect, the sheet material of the first region is formed of a smooth surface.

According to a fourteenth aspect, in the overtube of any one of the sixth to thirteenth aspects, at least a part of any one of the first contact surface or the second contact surface includes a high friction surface.

According to a fifteenth aspect, in the overtube of any one of the sixth to fourteenth aspects, the interlayer is provided between the outer tube and the shape-deformable body.

According to a sixteenth aspect, in the overtube of any one of the first to fifteenth aspects, the shape-deformable body includes a spiral tube formed by spirally winding a strip-shaped member on an outer peripheral side of the inner tube.

According to a seventeenth aspect, in the overtube of any one of the first to sixteenth aspects, a length of the second region in a longitudinal direction is 5 cm or more and 20 cm or less, and a proximal end of the second region is provided at a position within 20 cm from a distal end.

According to an eighteenth aspect, in the overtube of any one of the first to seventeenth aspects, a length of the first region in a longitudinal direction is longer than a length of the second region.

According to a nineteenth aspect, in the overtube of any one of the first to eighteenth aspects, the length of the first region in the longitudinal direction is 20 cm or more.

According to a twentieth aspect, in the overtube of any one of the third or ninth to eleventh aspects, a length of the second region in a longitudinal direction is 5 cm or more and 50 cm or less, and a proximal end of the second region is provided at a position within 50 cm from a distal end.

According to a twenty-first aspect, there is provided an insertion method of an endoscope insertion part using the overtube of any one of the first to twentieth aspects, the insertion method comprising: an insertion step of inserting the endoscope insertion part and the overtube from a mouth to a stomach of a subject in a state in which the endoscope insertion part is inserted into the overtube; an alignment step of performing alignment at a position where a distal end part of the endoscope insertion part is exposed from a distal end of the overtube; and a pylorus passage step of pushing the endoscope insertion part and the overtube together to cause the endoscope insertion part and the overtube to pass through a pylorus, after the insertion step and the alignment step are performed.

The insertion method of an endoscope insertion part according to a twenty-second aspect is the insertion method according to the twenty-first aspect, further comprising a retraction step of retracting the endoscope insertion part and the overtube together and moving a part of the overtube to a small bulge portion side to reduce slack of the overtube.

The insertion method of an endoscope insertion part according to a twenty-third aspect is the insertion method according to the twenty-first or twenty-second aspect, further comprising a stiffening step of stiffening the overtube.

According to a twenty-fourth aspect, there is provided an insertion method of an endoscope insertion part using the overtube of any one of the first to twentieth aspects, the insertion method comprising: an insertion step of inserting the endoscope insertion part and the overtube from a mouth to a stomach of a subject in a state in which the endoscope insertion part is inserted into the overtube; an endoscope pylorus passage step of pushing the endoscope insertion part to cause a part of at least a bendable part of the endoscope insertion part to pass through a pylorus, after the insertion step is performed; and an overtube pylorus passage step of pushing the overtube to cause the overtube to pass through the pylorus, after the endoscope pylorus passage step is performed.

The insertion method of an endoscope insertion part according to a twenty-fifth aspect is the insertion method according to the twenty-fourth aspect, further comprising an alignment step of performing alignment at a position where a distal end part of the endoscope insertion part is exposed from a distal end of the overtube, after overtube pylorus passage step is performed.

The insertion method of an endoscope insertion part according to a twenty-sixth aspect is the insertion method according to the twenty-fourth or twenty-fifth aspect, further comprising a retraction step of retracting the endoscope insertion part and the overtube together and moving a part of the overtube to a small bulge portion side to reduce slack of the overtube.

The insertion method of an endoscope insertion part according to a twenty-seventh aspect is the insertion method according to any one of the twenty-fourth to twenty-sixth aspects, further comprising a stiffening step of stiffening the overtube.

The insertion method of an endoscope insertion part according to a twenty-eighth aspect is the insertion method according to the twenty-seventh aspect, further comprising an insertion step of pushing the endoscope insertion part into the overtube to insert the endoscope insertion part into a deep portion inside a body, after the stiffening step is performed.

According to the present invention, it is possible to improve the insertability of the distal end side of the overtube into the digestive tract.

### BRIEF DESCRIPTION OF THE DRAWINGS

Fig. 1 is a schematic view showing a configuration of an endoscope apparatus.
Fig. 2 is a schematic cross-sectional view showing a configuration of a first region of an overtube.
Fig. 3 is a schematic cross-sectional view showing a configuration of a second region of the overtube.
Fig. 4 is a view showing a state in which the overtube is pushed into an endoscope insertion part.
Fig. 5 is a flowchart showing a procedure of a first example of a pylorus passage technique.
Fig. 6 is an explanatory view for explaining a first example of the pylorus passage technique.
Fig. 7 is a flowchart showing a procedure of a second example of the pylorus passage technique.
Fig. 8 is an explanatory view for explaining a second example of the pylorus passage technique.
Fig. 9 is a schematic cross-sectional view showing a configuration of an overtube of a second embodiment.
Fig. 10 is a view in which a sheet material provided in the overtube of the second embodiment is developed in a planar shape.
Fig. 11 is an external view of the overtube of the second embodiment in which an outer tube is omitted.
Fig. 12 is a view in which a sheet material according to a first modification example is developed in a planar shape.
Fig. 13 is a view in which a sheet material according to a second modification example is developed in a planar shape.
Fig. 14 is a view in which a sheet material according to a third modification example is developed in a planar shape.
Fig. 15 is a view in which a sheet material according to a fourth modification example is developed in a planar shape.
Fig. 16 is a view in which a sheet material according to a fifth modification example is developed in a planar shape.
Fig. 17 is a view in which a sheet material according to a sixth modification example is developed in a planar shape.
Fig. 18 is a view showing an evaluation result of a degree of curvature of the overtube.
Fig. 19 is a graph showing a result of measuring a stiffness of the second region of the overtube.
Fig. 20 is a view showing another configuration example of the overtube of the first embodiment.

### DESCRIPTION OF THE PREFERRED EMBODIMENTS

Hereinafter, embodiments of the present invention will be described with reference to the accompanying drawings.

### First Embodiment

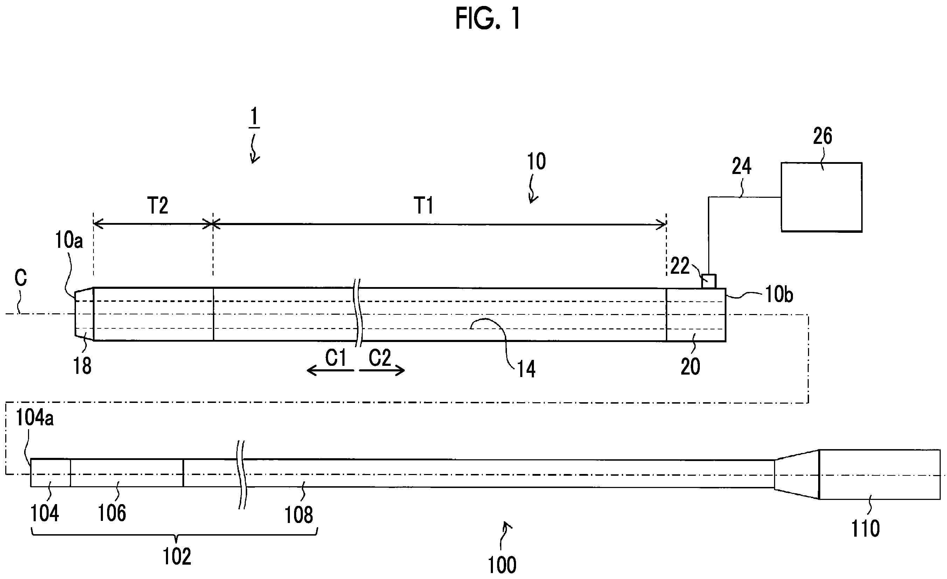

Fig. 1 is a schematic view showing a configuration of an endoscope apparatus 1. As shown in Fig. 1, the endoscope apparatus 1 comprises an overtube 10 and an endoscope 100. Hereinafter, before a specific configuration of the overtube 10 to which the present invention is applied will be described, a configuration of the endoscope 100 will be briefly described.

### Endoscope

The endoscope 100 comprises an elongated insertion part 102 (hereinafter, referred to as an "endoscope insertion part 102") to be inserted into a body, and a hand operating part 110 continuously provided on a proximal end side of the endoscope insertion part 102.

The endoscope insertion part 102 is configured to include a distal end part 104, a bendable part 106, and a soft part 108 in order from a distal end side to a proximal end side.

Although not shown, a pair of illumination windows for illuminating the inside of the body, an observation window for observing the inside of the body, a treatment tool outlet port for leading out a treatment tool, and a washing nozzle for washing the observation window are provided on a distal end surface 104a of the distal end part 104.

The bendable part 106 is operated to be bent in a desired direction by operating a pair of angle knobs (not shown) provided in the hand operating part 110. The soft part 108 is formed of a flexible member having a flexibility in a bending direction. It should be noted that the configuration of the endoscope 100 is not directly related to the present invention, and a known configuration is applied to the endoscope 100. Therefore, the description thereof will be omitted.

### Overtube

Next, the configuration of the overtube 10 will be described. The overtube 10 is a device that guides the endoscope insertion part 102 into the body, and is a variable stiffness overtube of which a stiffness is variable. The endoscope insertion part 102 is an example of a medical instrument according to the embodiment of the present invention.

As shown in Fig. 1, the overtube 10 is an elongated member formed along a longitudinal axis C extending from a distal end 10a to a proximal end 10b. In a direction along the longitudinal axis C, one side (the distal end 10a side) is referred to as a distal end direction (a direction of a reference C1 in Fig. 1) or a distal end side, and the other side (the proximal end 10b side), which is directed to an opposite side of the one side, is referred to as a proximal end direction (a direction of a reference C2 in Fig. 1) or a proximal end side.

An insertion path 14 provided along the longitudinal axis C is provided inside the overtube 10. The insertion path 14 is open to both the distal end 10a and the proximal end 10b of the overtube 10, and communicates between a distal end-side opening (distal end opening) and a proximal end-side opening (proximal end opening). An inner diameter of the insertion path 14 is larger than an outer diameter of the endoscope insertion part 102 and is formed, and the endoscope insertion part 102 can be inserted into the insertion path 14.

A distal end cap 18 is provided on the distal end side of the overtube 10, and a proximal end cap 20 is provided on the proximal end side of the overtube 10.

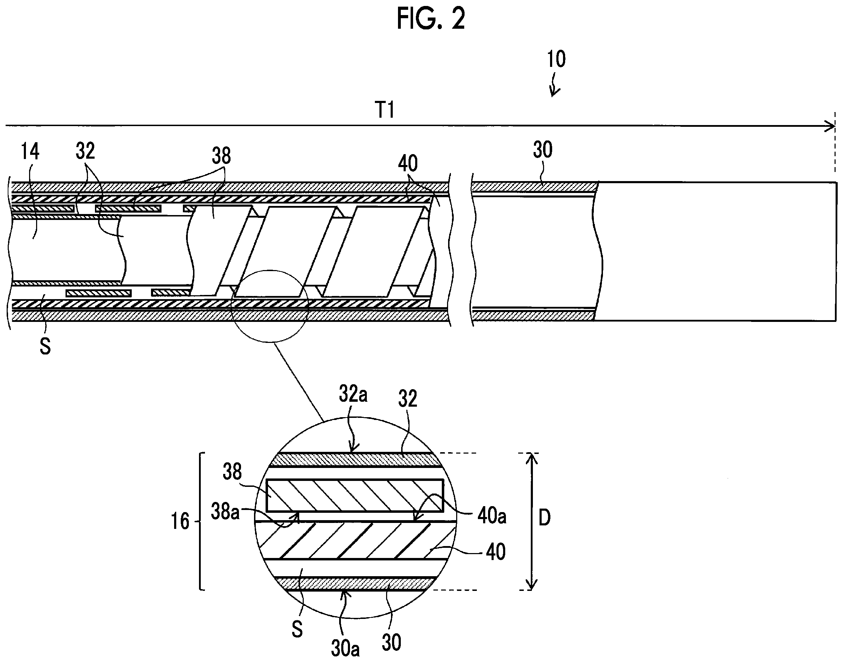

A portion of the overtube 10 excluding the distal end cap 18 and the proximal end cap 20 (hereinafter, referred to as a "tube body"), that is, a region that occupies a major part excluding both end portions (portions on the distal end side and the proximal end side) in a direction along the longitudinal axis C is configured in a circular tubular shape elongated along the longitudinal axis C. Although the details will be described later, the tube body constituting the overtube 10 is formed of a double-wall structure tube in which an outer tube 30 covers a periphery of an inner tube 32, and a space S (hereinafter, referred to as a "tube inner space S") is provided between the outer tube 30 and the inner tube 32 (see Fig. 2).

The distal end cap 18 is a member that constitutes the distal end side of the overtube 10. The distal end cap 18 is a tubular body made of a rigid resin that is fixed coaxially to a distal end side of the tube body, and constitutes the distal end-side opening of the overtube 10 and a part of the insertion path 14. In addition, the distal end cap 18 also serves as a sealing member for sealing an end portion of the tube inner space S on the distal end side.

The proximal end cap 20 is a member that constitutes the proximal end side of the overtube 10. The proximal end cap 20 is a tubular body made of a rigid resin that is fixed coaxially to a proximal end side of the tube body, and constitutes the proximal end-side opening of the overtube 10 and a part of the insertion path 14. In addition, the proximal end cap 20 also serves as a sealing member for sealing an end portion of the tube inner space S on the proximal end side.

The proximal end cap 20 has a predetermined length (at least a length that can be gripped by an operation person such as an operator or an assistant) along the longitudinal axis C, and functions as a grip portion to be gripped by the operation person.

A supply and discharge port 22 is provided on an outer peripheral surface of the proximal end cap 20. The supply and discharge port 22 communicates with the tube inner space S through a communication pipe line (not shown) provided inside an outer peripheral wall of the proximal end cap 20. The supply and discharge port 22 is connected to a pump 26 via a fluid supply and discharge tube 24. Accordingly, by operating the pump 26, it is possible to supply and discharge a fluid (in the present example, air) to and from the tube inner space S through the supply and discharge port 22. The fluid to be sent into the tube inner space S is not limited to air. Various other gases may be used, and a liquid may be used.

The overtube 10 has a first region T1 located on the distal end side with respect to the supply and discharge port 22 and a second region T2 located on the distal end side with respect to the first region T1. Specifically, the second region T2 is a region having a predetermined length from the distal end (proximal end of the distal end cap 18) of the tube body constituting the overtube 10 toward the proximal end direction C2. The first region T1 is a region from a proximal end of the second region T2 to the proximal end (distal end of the proximal end cap 20) of the tube body.

The distal end cap 18 may not necessarily be provided on the distal end side of the overtube 10. In this case, the tube inner space S is sealed on the distal end side of the tube body. In a case where the distal end cap 18 is not provided, the distal end 10a of the overtube 10 substantially coincides with a distal end of the second region T2.

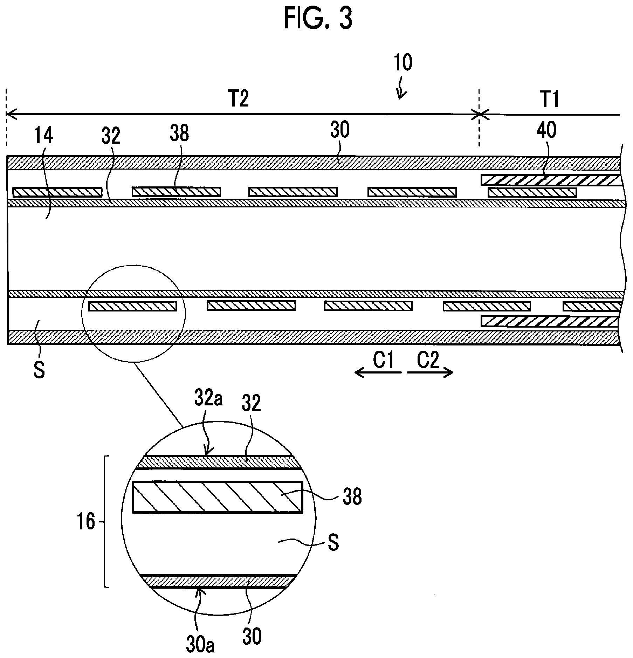

Next, configurations of the first region T1 and the second region T2 of the overtube 10 will be described in detail. Fig. 2 is a schematic cross-sectional view showing the configuration of the first region T1 of the overtube 10. Fig. 3 is a schematic cross-sectional view showing the configuration of the second region T2 of the overtube 10. In Fig. 3, a part of the first region T1 is also shown in order to show a difference between the first region T1 and the second region T2. In the following, configurations common to the first region T1 and the second region T2 will be described, and then configurations different between the first region T1 and the second region T2 will be described.

As shown in Figs. 2 and 3, the tube body constituting the overtube 10 has a cylindrical tube outer peripheral wall 16 extending from the distal end to the proximal end. The tube outer peripheral wall 16 is formed of a double-wall structure tube in which the outer tube 30 covers the periphery of the inner tube 32. That is, the tube body includes the flexible outer tube 30 and the flexible inner tube 32 disposed inside the outer tube 30, and the tube inner space S is provided between the outer tube 30 and the inner tube 32. A shape-deformable body 38 and a sheet material 40, which will be described later, are disposed in the tube inner space S. As will be described later, in the present embodiment, the sheet material 40 is disposed only in the first region T1.

The outer tube 30 and the inner tube 32 are each formed of, for example, a soft resin material such as urethane or polyester resin. The outer tube 30 and the inner tube 32 are not limited to the above-described examples, and a material having an elastic modulus of about 5 MPa (megapascal, the same applies hereinafter) to 20 MPa at an elongation rate of 100% (percent, the same applies hereinafter) obtained by a method described in JISK7161 (tensile test) or a method based thereon can be applied.

A thickness of each of the outer tube 30 and the inner tube 32 is, for example, about 100 µm (micrometers, the same applies hereinafter) and 200 µm. In addition, from the viewpoint of preventing breakage, it is preferable that the thickness of the outer tube 30 is larger than the thickness of the inner tube 32. A rigidity of each of the outer tube 30 and the inner tube 32 is smaller than that of the sheet material 40 described below.

The tube inner space S is a space in which a cross-sectional shape in a direction orthogonal to the longitudinal axis C is formed in an annular (donut) shape so as to surround the periphery (outer periphery) of the inner tube 32. The tube inner space S is formed as a sealed space by sealing the outer tube 30 and the inner tube 32 on the distal end side and the proximal end side of the overtube 10 by the distal end cap 18 and the proximal end cap 20 described above.

A thickness of the tube inner space S is about 600 µm to 800 µm, for example, in a case where a thickness D of the tube outer peripheral wall 16 in the overtube 10 in a softened state is 1 mm (millimeter, the same applies hereinafter) or less. The thicknesses of the outer tube 30, the inner tube 32, and the tube inner space S are not limited to the above-mentioned thicknesses, and are set based on a diameter of the endoscope insertion part 102, a diameter of an insertion passage of a target into which the endoscope insertion part 102 is inserted, and the like. In addition, an inner diameter of the inner tube 32 (that is, an inner diameter of the insertion path 14) is also set according to the outer diameter of the endoscope insertion part 102, and the inner diameter is, for example, about 6 mm to 16 mm.

An outer peripheral surface 30a of the outer tube 30 and an inner peripheral surface 32a of the inner tube 32 each have a hydrophilic coating. By forming the hydrophilic coating on the outer peripheral surface 30a of the outer tube 30, a frictional resistance between the outer tube 30 and a digestive tract wall can be reduced. As a result, insertion ability (advancement ability) of the overtube 10 into the body is improved. In addition, by forming the hydrophilic coating on the inner peripheral surface 32a of the inner tube 32, a frictional resistance between the inner tube 32 and the endoscope insertion part 102 can be reduced. As a result, the insertion ability of the endoscope insertion part 102 into the overtube 10 is improved.

In the present example, an aspect in which the hydrophilic coating is formed on both the outer tube 30 and the inner tube 32 has been shown, but the present invention is not limited thereto. The hydrophilic coating may be formed on at least one of the outer tube 30 or the inner tube 32. However, it is preferable to form the hydrophilic coating on both the outer tube 30 and the inner tube 32 because the above-described two effects can be obtained.

A shape-deformable body 38 is provided between the outer tube 30 and the inner tube 32 (that is, in the tube inner space S). As shown in Figs. 2 and 3, the shape-deformable body 38 is provided from the distal end to the proximal end of the tube body constituting the overtube 10 and is disposed in each of the first region T1 and the second region T2. The shape-deformable body 38 is formed of a spiral pipe formed by spirally winding a strip-shaped member on the outer peripheral side of the inner tube 32 along the longitudinal axis C. The shape-deformable body 38 has a flexibility in a bending direction and can be deformed following the shape of the overtube 10. For example, in a case where the overtube 10 in a softened state is deformed into a curved shape or any other shape, the shape-deformable body 38 is also deformed following the shape.

The strip-shaped member constituting the spiral pipe is made of stainless steel (steel use stainless (SUS)). The strip-shaped member is not limited to being made of stainless steel, and a material that can be deformed following the shape of the overtube 10 can be applied. For example, the strip-shaped member may be formed of plastic or the like. A thickness of the strip-shaped member is preferably 300 µm or less, for example, in a case where the thickness D of the tube outer peripheral wall 16 in the overtube 10 in a softened state is 1 mm or less.

Next, the configurations different between the first region T1 and the second region T2 will be described. As shown in Figs. 2 and 3, the overtube 10 in the present embodiment has different configurations between the first region T1 and the second region T2. In the following, the configuration of the first region T1 will be described, and then the configuration of the second region T2 will be described.

The first region T1 is a region located on a proximal end side of the second region T2. A length of the first region T1 in a longitudinal direction (a direction along the longitudinal axis C, the same applies hereinafter) is longer than a length of the second region T2 in the longitudinal direction. Specifically, the length of the first region T1 in the longitudinal direction is at least 20 cm (centimeters, the same applies hereinafter) or more, and is, for example, 20 cm or more and 100 cm or less.

The first region T1 is switchable between a softened state (also referred to as a flexible state or a non-stiffened state; corresponding to a "first state" of the present invention) in which air (an example of a "fluid") is supplied to the tube inner space S between the inner tube 32 and the outer tube 30 via the supply and discharge port 22 and a "second state" (also referred to as a "stiffened state" or a "stiff state"; corresponding to a "second state" according to the present invention) which is stiffer than the first state and in which the air is discharged from the tube inner space S). Specifically, the first region T1 has the following configuration.

As shown in Fig. 2, the first region T1 has the outer tube 30, the inner tube 32, and the shape-deformable body 38 as described above. The sheet material 40 is provided in the first region T1. The sheet material 40 is an interlayer disposed between the outer tube 30 and the inner tube 32, and is disposed to be contactable with the shape-deformable body 38. Specifically, the sheet material 40 is interposed between the outer tube 30 and the shape-deformable body 38 and has a cylindrical shape so as to surround the shape-deformable body 38. In addition, the sheet material 40 is provided over the entire first region T1 in the longitudinal direction. As will be described later, in the present embodiment, the sheet material 40 is not provided in the second region T2. The sheet material 40 has a flexibility to be deformable following the shape of the overtube 10.

The sheet material 40 is made of, for example, a resin such as urethane. However, the present invention is not limited to this, and for example, an aluminum-deposited film can also be applied. The aluminum-deposited film is a film in which aluminum (aluminum foil) is vacuum-deposited (thermocompression-bonded) on a surface of a substrate film. By applying the aluminum-deposited film as the sheet material 40, a rigidity of the sheet material 40 can be increased. In this case, examples of the substrate film include a substrate film having heat sealability such as polyethylene terephthalate (PET) or polyethylene (PE). In addition, it is preferable that an elastic modulus of the sheet material 40 is higher than that of the outer tube 30 and the inner tube 32. The elastic modulus of the sheet material 40 is obtained by the method described in JISK7161 (tensile test) or the method based thereon. Thus, the outer tube 30 and the inner tube 32 can be reinforced by the sheet material 40. That is, a tensile strength and a tensile rigidity of the overtube 10 are increased by the sheet material 40. As a result, the advancement ability of the overtube 10 in a softened state is improved. A material having an optimum elastic modulus can be selected by using the aluminum-deposited film or aluminum foil combined with various kinds of cloth and film.

A thickness of the sheet material 40 is preferably 300 µm or less, for example, in a case where the thickness of the tube outer peripheral wall 16 of the overtube 10 in a softened state is 1 mm or less. However, from the viewpoint of a rigidity, the sheet material 40 is preferably thicker than the thicknesses of the outer tube 30 and the inner tube 32. In addition, in a case where the aluminum-deposited film is applied as the sheet material 40, the rigidity of the overtube 10 can be changed by changing the substrate film. As a result, the stiffness of the overtube 10 in a softened state can be tuned to a stiffness suitable for an insertion site (an upper digestive tract or a lower digestive tract) of the endoscope insertion part 102.

The sheet material 40 has a first contact surface 40a on a side (inner peripheral surface side) facing the shape-deformable body 38. The first contact surface 40a is a surface that can come into contact with the shape-deformable body 38, and the entire first contact surface 40a is formed of a high friction surface. That is, the sheet material 40 of the present example is formed of a friction sheet material having a high friction surface on a surface (first contact surface 40a) on a side facing the shape-deformable body 38. Accordingly, the first contact surface 40a of the sheet material 40 comes into contact with the shape-deformable body 38 with high friction, so that it is possible to restrict the movement of the first contact surface 40a in a direction along the longitudinal axis C of the shape-deformable body 38 and to increase a shape retention force of the shape-deformable body 38.

The high friction surface is formed of, for example, a resin layer formed by coating an inner peripheral surface of the sheet material 40 with a resin such as a urethane coating or a silica coating. In addition, the high friction surface may be formed of a rough surface in which fine unevenness is formed on the inner peripheral surface of the sheet material 40. The rough surface is formed by performing a surface roughening treatment such as blasting, laser irradiation, a chemical conversion treatment, or etching. The high friction surface may be a surface obtained by coating the roughened inner peripheral surface of the sheet material 40 with the above-described resin or a surface obtained by roughening the inner peripheral surface of the sheet material 40 coated with the above-described resin.

Here, in the present specification, the "high friction surface" refers to a surface having a higher friction coefficient than a surface (in the present example, an inner peripheral surface of the outer tube 30) in contact with the shape-deformable body 38 in a case where the sheet material 40 is not interposed. That is, the "high frictional surface" generates a frictional resistance higher than a frictional resistance in a case where the shape-deformable body 38 and the outer tube 30 are in direct contact with each other without the sheet material 40 being interposed. The friction coefficient of the "high friction surface" can be measured, for example, by a method described in JISP8147:2010 or a method based thereon.

In addition, in the present specification, "non-deformable" means that the shape of the shape-deformable body 38 is fixed in a case where the air in the tube inner space S is discharged. In addition, in the present specification, "frictional engagement" means that contact surfaces in contact with each other engage with each other by a frictional force. This frictional force is a frictional force (static frictional force) generated in a case where the contact surfaces come into contact with each other in a radial direction of the overtube 10 in a case where the overtube 10 is viewed from a direction along the longitudinal axis C. In addition, each of the contact surfaces is formed in a substantially circular shape as viewed in a direction of the longitudinal axis C.

In the present embodiment, as an example, an aspect in which the first contact surface 40a of the sheet material 40 on the side (inner peripheral surface side) facing the shape-deformable body 38 is formed of a high friction surface has been illustrated. However, the present disclosure is not limited thereto. A second contact surface 38a of the shape-deformable body 38 on a side (outer peripheral surface side) facing the sheet material 40 may be formed of a high friction surface. In addition, both the first contact surface 40a of the sheet material 40 and the second contact surface 38a of the shape-deformable body 38 may be formed of a high friction surface. That is, it is sufficient that at least one of the first contact surface 40a of the sheet material 40 or the second contact surface 38a of the shape-deformable body 38 is formed of a high friction surface.

In addition, in the present embodiment, the high friction surface may be formed on an entirety of at least one of the first contact surface 40a or the second contact surface 38a, or may be formed on a part of any one of the first contact surface 40a or the second contact surface 38a. That is, the high friction surface may be formed on at least a part of any one of the contact surfaces. However, from a viewpoint of increasing a shape retention force of the shape-deformable body 38, an aspect in which the high friction surface is formed on an entirety of any one of the contact surface is preferable, and an aspect in which the high friction surface is formed on an entirety of both the contact surfaces is more preferable. In addition, from a viewpoint of ensuring a flexibility of the overtube 10 in a softened state, a gap between the shape-deformable body 38 and the sheet material 40 is preferably about 50 µm to 500 µm.

In addition, the sheet material 40 according to the present embodiment is formed in a sheet shape by a smooth surface in which a cut portion such as a cut hole or a cut line is not provided, but the present disclosure is not limited to this. The sheet material 40 may be provided with a cut portion as long as a stiffness of the first region T1 in the overtube 10 is stiffer than a stiffness of the second region T2. In addition, the sheet material 40 is not limited to one sheet material, and may be composed of a plurality of sheet materials.

Next, the configuration of the second region T2 will be described.

As shown in Fig. 3, the second region T2 is the same as the first region T1 in that the outer tube 30, the inner tube 32, and the shape-deformable body 38 are provided. The second region T2 is different from the first region T1 in that the sheet material 40 is not interposed between the outer tube 30 and the shape-deformable body 38. As a result, the second region T2 is configured as a region softer than the first region T1.

That is, in the present embodiment, in order to configure the second region T2 in the overtube 10 as a region softer than the first region T1, the overtube 10 has a configuration in which the sheet material 40 is interposed between the outer tube 30 and the shape-deformable body 38 in the first region T1 and the sheet material 40 is not interposed between the outer tube 30 and the shape-deformable body 38 in the second region T2.

In addition, in the same manner as the first region T1, the second region T2 is switchable between a softened state (corresponding to a "first state" of the present invention) in which air is supplied to the tube inner space S between the inner tube 32 and the outer tube 30 via the supply and discharge port 22 and a stiffened state (corresponding to a "second state" of the present invention) which is a state in which air is discharged from the tube inner space S and which is stiffer than the softened state. However, the second region T2 is configured such that the stiffness of the second region T2 in each state is softer than the stiffness of the first region T1 in each corresponding state due to a difference in disposition of the sheet material 40 described above.

Specifically, in a case where the overtube 10 is in a stiffened state (that is, in a second state) (that is, in a case where the first region T1 and the second region T2 are in a stiffened state), the second region T2 is configured to be softer than the first region. In addition, in a case where the overtube 10 is in a softened state (that is, in a first state) (that is, in a case where the first region T1 and the second region T2 are in a softened state), the second region T2 is configured to be softer than the first region.

With such a configuration, the second region T2 is configured as a region softer than the first region T1 in any of a stiffened state and a softened state of the overtube 10. Therefore, for example, even in a case where the endoscope insertion part 102 is subjected to a bending operation in a state in which the endoscope insertion part 102 is inserted into the overtube 10 in a stiffened state, since the second region T2 is configured as a region softer than the first region T1, the bending operation is not significantly hindered by the overtube 10 in a stiffened state, and a sufficient amount of curvature can be obtained.

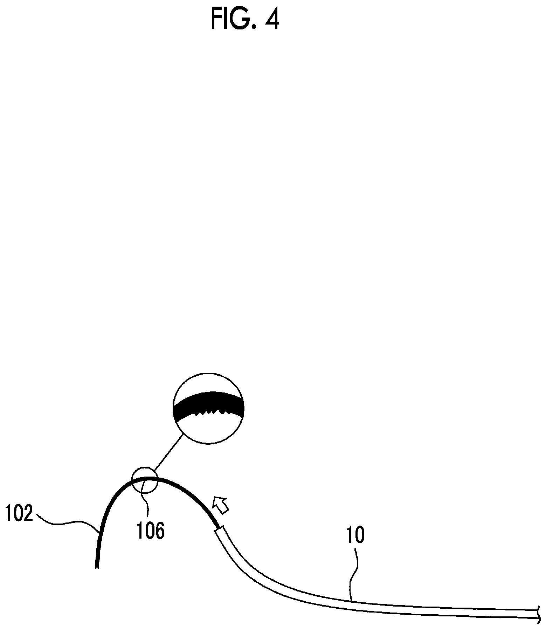

In addition, in a case where the overtube 10 in a softened state and the endoscope insertion part 102 are operated to advance and retreat relative to each other, for example, as shown in Fig. 4, even in a case where an amount of curvature of the endoscope insertion part 102 led out from the distal end of the overtube 10 is large, since the second region T2 is configured as a region softer than the first region T1, it is possible to smoothly push the overtube 10 along a wall surface of the digestive tract without being caught on wrinkles or the like generated in the bendable part 106 of the endoscope insertion part 102.

Here, a length of the second region T2 in the longitudinal direction is preferably 5 cm or more and 20 cm or less (more preferably 7 cm or more and 15 cm or less). In addition, the proximal end of the second region T2 is preferably provided at a position within 20 cm from the distal end 10a of the overtube 10. It is preferable that the length and the position described above are determined based on an angle length (a length from a distal end of the endoscope insertion part 102 to a proximal end of the bendable part 106) of the endoscope insertion part 102 used in combination with the overtube 10. The angle length of the endoscope insertion part 102 is, for example, 7 cm in an upper endoscope and 10 cm in a lower endoscope.

By configuring the second region T2 to have the above-described length and position, in a case where the distal end 10a of the overtube 10 and the distal end of the endoscope insertion part 102 are matched in a state in which the endoscope insertion part 102 is inserted into the overtube 10, the second region T2 is disposed at least in a region in which the bendable part 106 of the endoscope insertion part 102 is disposed. Accordingly, even in a case where the endoscope insertion part 102 is retracted into the overtube 10 so that the distal end 10a of the overtube 10 and the distal end of the endoscope insertion part 102 are substantially matched, the second region T2 is present at a position corresponding to the bendable part 106 of the endoscope insertion part 102. Therefore, even in a case where the endoscope insertion part 102 is subjected to a bending operation in a state in which the overtube 10 is overlapped with the bendable part 106 of the endoscope insertion part 102, the overtube 10 is not significantly hindered by the bending operation, and thus a sufficient amount of curvature can be obtained and the endoscope insertion part 102 can be guided in a desired direction.

According to the overtube 10 configured as described above, in the first region T1, in a case where the air in the tube inner space S is discharged by the pump 26, the inner peripheral surface of the outer tube 30 is pressed against the shape-deformable body 38 via the sheet material 40 to be in close contact with the shape-deformable body 38, and the outer peripheral surface of the inner tube 32 is pressed against the shape-deformable body 38 to be in close contact with the shape-deformable body 38, so that the position of the shape-deformable body 38 is fixed. As a result, the first region T1 changes from the softened state (first state) to the stiffened state (second state) stiffer than the softened state. In this case, the shape-deformable body 38 and the sheet material 40 are in close contact with each other via a high friction surface and are frictionally engaged with each other. As a result, the movement of the shape-deformable body 38 in a direction along the longitudinal axis C is restricted, and the shape (for example, the curved shape) of the shape-deformable body 38 is held to be non-deformable. Therefore, a shape retention force of the first region T1 in the stiffened state is increased. On the contrary, in a case where air flows into the tube inner space S from a state in which the air is discharged, the shape retention of the shape-deformable body 38 is released, so that the first region T1 changes from the stiffened state to the softened state.

On the other hand, in the second region T2, in a case where the air in the tube inner space S is discharged by the pump 26, the inner peripheral surface of the outer tube 30 is pressed against the shape-deformable body 38 to be in close contact with the shape-deformable body 38, and the outer peripheral surface of the inner tube 32 is pressed against the shape-deformable body 38 to be in close contact with the shape-deformable body 38, so that the position of the shape-deformable body 38 is fixed. As a result, the second region T2 changes from the softened state (first state) to the stiffened state (second state) stiffer than the softened state. In this case, since the sheet material 40 is not provided in the second region T2, the second region T2 changed to the stiffened state is softer than the first region T1 in the stiffened state. On the contrary, in a case where air flows into the tube inner space S from a state in which the air is discharged, the second region T2 changes from the stiffened state (second state) to the softened state (first state). Even in the second region T2 in the softened state, since the sheet material 40 is not provided, the second region T2 is softer than the softened state of the first region T1.

As described above, in any of the stiffened state and the softened state the state, the second region T2 is configured to be softer than the first region T1 in each of corresponding states (that is, the stiffened state and the softened state). Therefore, the insertability of the distal end side of the overtube 10 into the digestive tract (particularly, an upper digestive tract) is improved. Accordingly, in any state, the endoscope insertion part 102 can be stably held by the first region T1 and the endoscope insertion part 102 can be guided in a desired direction by the second region T2. As a result, it is possible to easily perform an operation of moving the overtube 10 and the endoscope insertion part 102 inside the body in a case where a pylorus passage technique, which will be described later, is performed.

### Pylorus Passage Technique

As an example of a technique using the overtube 10 according to this embodiment, a technique for passing the endoscope insertion part 102 through a pylorus between a stomach and a small intestine (pylorus passage technique) will be described. The pylorus passage technique is an example, and other techniques partially including the same operations as the pylorus passage technique can be realized by combining the same procedures as the pylorus passage technique.

### First Example of Pylorus Passage Technique

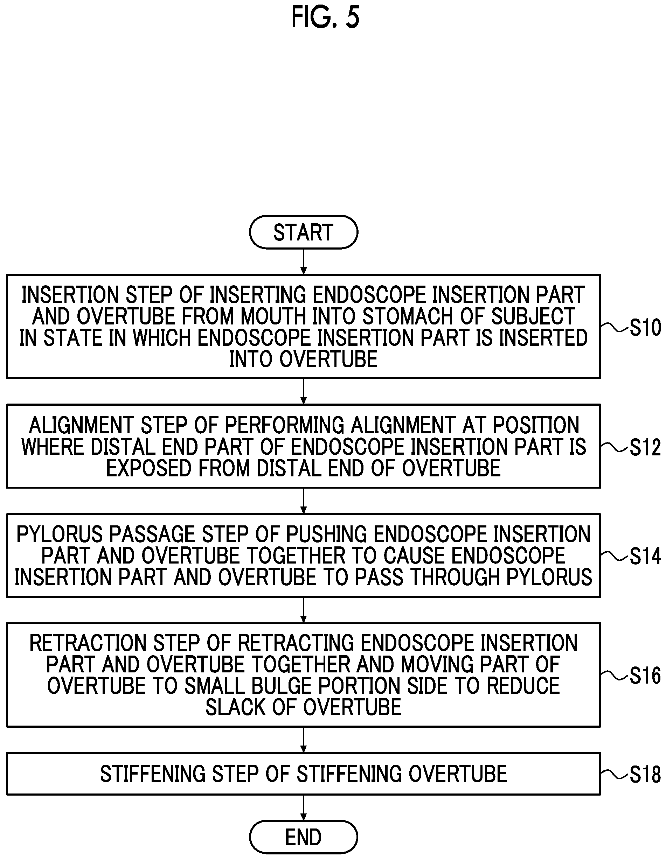

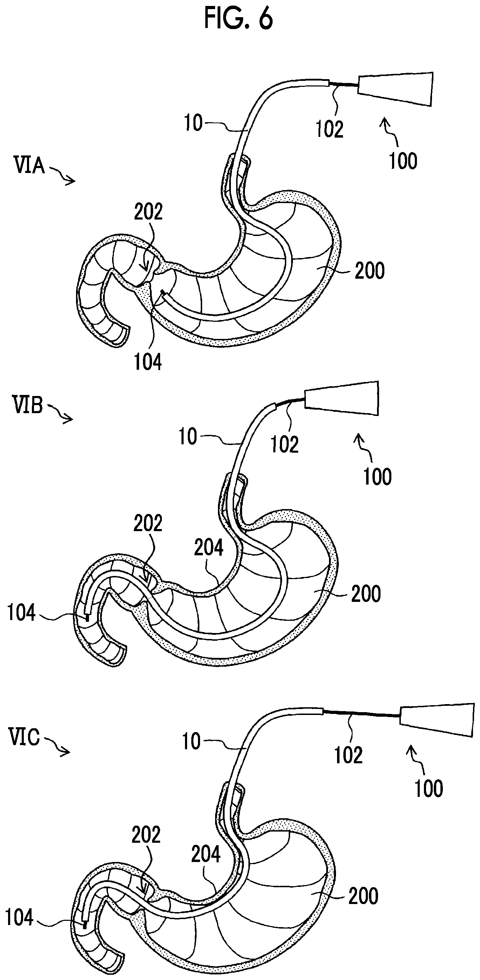

The first example of the pylorus passage technique will be described with reference to Figs. 5 and 6. Fig. 5 is a flowchart showing a procedure of the first example of the pylorus passage technique. Fig. 6 is an explanatory view for explaining the first example of the pylorus passage technique. In addition, at the start of this flowchart, it is assumed that the overtube 10 is in a softened state. The same applies to a second example described later.

### Step S10: Insertion Step

First, in a state in which the endoscope insertion part 102 is inserted into the overtube 10, the endoscope insertion part 102 and the overtube 10 are inserted from a mouth into a stomach 200 of a subject.

### Step S12: Alignment Step

Next, as shown in VIA of Fig. 6, alignment is performed at a position where the distal end part 104 of the endoscope insertion part 102 is exposed from the distal end opening of the overtube 10. This alignment is performed by moving the endoscope insertion part 102 in the proximal end direction C2 (proximal side) with respect to the overtube 10 or by moving the overtube 10 in the distal end direction C1 (distal side) with respect to the endoscope insertion part 102.

An insertion step may be performed after the alignment step. By performing the insertion step after performing the alignment step, the overtube 10 and the endoscope insertion part 102 can be inserted into the stomach 200 while obtaining a forward visual field in the insertion direction by the illumination window and the observation window at the distal end of the endoscope insertion part 102.

### Step S14: Pylorus Passage Step

Next, as shown in VIB of Fig. 6, the endoscope insertion part 102 and the overtube 10 are pushed together, and the endoscope insertion part 102 and the overtube 10 are made to pass through a pylorus 202. Specifically, the endoscope insertion part 102 and the overtube 10 are pushed together while the bendable part 106 is operated to be bent such that the distal end part 104 of the endoscope insertion part 102 is guided toward the pylorus 202. In this case, since the second region T2 of the overtube 10 in the softened state is configured as a region softer than the first region T1, the second region T2 can be bent to follow the bending operation of the endoscope insertion part 102. Therefore, since the bending operation of the endoscope insertion part 102 is not significantly hindered by the second region T2 (that is, since a degree of freedom of the bending operation of the endoscope insertion part 102 is not significantly limited), the endoscope insertion part 102 and the overtube 10 can be pushed together while guiding the distal end part 104 of the endoscope insertion part 102 toward the pylorus 202 by the bending operation of the endoscope insertion part 102. As a result, it is possible to allow the endoscope insertion part 102 and the overtube 10 to easily and stably pass through the pylorus 202.

### Step S16: Retraction Step

Next, as shown in VIC of Fig. 6, the endoscope insertion part 102 and the overtube 10 are retracted together, and a part of the overtube 10 is moved to a small bulge portion 204 side to reduce slack of the overtube 10. Here, after the pyloric passage step is performed, as shown in VIB of Fig. 6, large slack occurs in a part (middle portion) of the overtube 10 inside the stomach 200. In a case where such a looseness occurs in the overtube 10, there may be a problem in a case where the overtube 10 and the endoscope insertion part 102 are further pushed into a deep portion inside the body. Therefore, it is desirable to keep the overtube 10 and the endoscope insertion part 102 in a state close to a straight line at this stage and to allow the overtube 10 and the endoscope insertion part 102 to be further pushed into the deep portion inside the body. Therefore, after the pylorus passage step is performed, the retraction step is performed to resolve the slack of the overtube 10 that occurs inside the stomach 200. As a result, the overtube 10 is in a state (stretched state) in which the slack of the overtube 10 inside the stomach 200 is resolved as compared to a state before the retraction step is performed.

### Step S18: Stiffening Step

Next, the overtube 10 is stiffened. Specifically, the pump 26 is operated to discharge the air in the tube inner space S through the supply and discharge port 22. Accordingly, in the first region T1, the outer tube 30 and the inner tube 32 are pressed against the shape-deformable body 38 that is deformed following the shape of the overtube 10. Then, the inner peripheral surface of the outer tube 30 is pressed against the shape-deformable body 38 to be in close contact therewith via the sheet material 40, and the outer peripheral surface of the inner tube 32 is pressed against the shape-deformable body 38 to be in close contact therewith, so that the position of the shape-deformable body 38 is fixed. As a result, the first region T1 changes from the softened state to the stiffened state. In this case, the shape-deformable body 38 and the sheet material 40 are in close contact with each other via a high friction surface and are frictionally engaged with each other. Accordingly, since the shape (for example, the curved shape) of the shape-deformable body 38 is held to be non-deformable, the first region T1 is stiffened in a shape corresponding to the shape-deformable body 38.

In addition, in a case where the pump 26 is operated to discharge the air in the tube inner space S through the supply and discharge port 22, in the second region T2, the inner peripheral surface of the outer tube 30 is pressed against the shape-deformable body 38 to be in close contact therewith, and the outer peripheral surface of the inner tube 32 is pressed against the shape-deformable body 38 to be in close contact therewith, so that the position of the shape-deformable body 38 is fixed. As a result, the second region T2 changes from the softened state to a stiffened state that is stiffer than the softened state. In this case, since the sheet material 40 is not provided in the second region T2, the second region T2 changed to the stiffened state is softer than the first region T1 in the stiffened state.

Therefore, in a case where the air in the tube inner space S is discharged through the supply and discharge port 22, in the overtube 10, the first region T1 is in a stiffened state, and the second region T2 located on the distal end side with respect to the first region T1 is in a state softer than the stiffened state of the first region T1. Accordingly, it is possible to hold the endoscope insertion part 102 in a stable state without wobbling inside the body by the first region T1, and it is possible to guide the endoscope insertion part 102 in a desired direction by the second region T2. As a result, the insertability of the endoscope insertion part 102 is improved.

Thereafter, the bendable part 106 of the endoscope insertion part 102 is caused to protrude forward from the distal end opening of the overtube 10, and a treatment tool (not shown) is caused to be led forward from the treatment tool outlet port of the distal end part 104 to start a treatment such as endoscopic submucosal dissection (ESD). In this case, since the insertability of the endoscope insertion part 102 is improved by the overtube 10 in a stiffened state, the distal end part 104 can be located at an accurate treatment position, and as a result, it is possible to perform an accurate treatment on a treatment target site (lesion portion or the like).

In the first example described above, a case where the treatment of the treatment target site is performed in a state in which the overtube 10 passing through the pylorus 202 is in a stiffened state only once has been described, but the present disclosure is not limited thereto. For example, an operation of performing a treatment on a treatment target site by advancing each of the endoscope insertion part 102 and the overtube 10 in a stepwise manner while changing the overtube 10 passing through the pylorus 202 between the softened state and the stiffened state a plurality of times may be performed. The operation in this case includes a step (endoscope insertion part advancing step) of advancing the endoscope insertion part 102 relative to the overtube 10 in a state in which the overtube 10 into which the endoscope insertion part 102 is inserted is in a stiffened state, and a step (overtube advancing step) of advancing the overtube 10 relative to the endoscope insertion part 102 in a state in which the overtube 10 into which the endoscope insertion part 102 is inserted is in a softened state. The same applies to a second example described later.

### Second Example of Pylorus Passage Technique

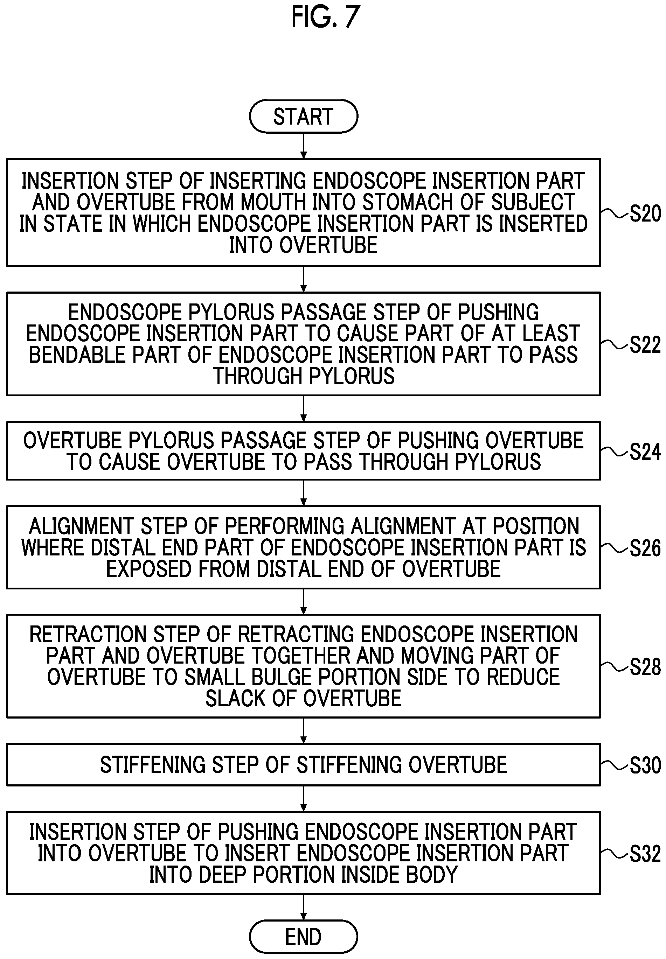

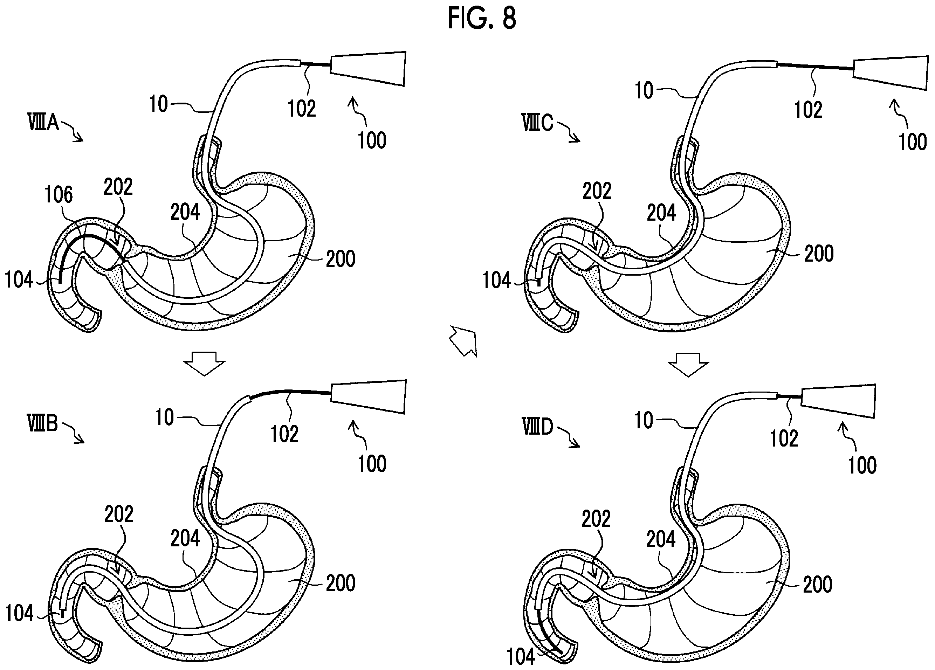

The second example of the pylorus passage technique will be described with reference to Figs. 7 and 8. Fig. 7 is a flowchart showing a procedure of the second example of the pylorus passage technique. Fig. 8 is a view for explaining the second example of the pylorus passage technique.

### Step S20: Insertion Step

First, in a state in which the endoscope insertion part 102 is inserted into the overtube 10, the endoscope insertion part 102 and the overtube 10 are inserted from the mouth into the stomach 200 of the subject.

### Step S22: Endoscope Pylorus Passage Step

Next, as shown in VIIIA of Fig. 8, the endoscope insertion part 102 is pushed, and a part of at least the bendable part 106 of the endoscope insertion part 102 is made to pass through the pylorus 202. In this case, the endoscope insertion part 102 is pushed while the bendable part 106 is operated to be bent such that the distal end of the endoscope insertion part 102 is guided toward the pylorus 202 while securing a visual field at the observation window and the illumination window at the distal end of the endoscope insertion part 102.

### Step S24: Overtube Pylorus Passage Step

Next, as shown in VIIIB of Fig. 8, the overtube 10 is pushed to pass through the pylorus 202. In this case, since the second region T2 of the overtube 10 in a softened state is configured as a region softer than the first region T1, even in a case where the overtube 10 is pushed along the endoscope insertion part 102 that has already passed through the pylorus 202, the overtube 10 can be smoothly pushed along the wall surface of the digestive tract without being caught on wrinkles or the like generated in the bendable part 106 of the endoscope insertion part 102 (see Fig. 4).

### Step S26: Alignment Step

Next, alignment is performed at a position where the distal end part 104 of the endoscope insertion part 102 is exposed from the distal end of the overtube 10. This alignment is performed by moving the endoscope insertion part 102 in the proximal end direction C2 (proximal side) with respect to the overtube 10 or by moving the overtube 10 in the distal end direction C1 (distal side) with respect to the endoscope insertion part 102. In the overtube pylorus passage step, in a case where the alignment between the distal end of the overtube 10 and the distal end part 104 of the endoscope insertion part 102 has already been made, the alignment step can be omitted. In addition, in a case where the endoscope insertion part 102 is inserted into the deep portion (duodenum) inside the body, the alignment step does not have to be performed.

### Step S28: Retraction Step

Next, as shown in VIIIC of Fig. 8, the endoscope insertion part 102 and the overtube 10 are retracted together, and a part of the overtube 10 is moved to the small bulge portion 204 side to reduce the slack of the overtube 10. As a result, as in the first example described above, the overtube 10 is in a state (stretched state) in which the slack of the overtube 10 inside the stomach 200 is resolved as compared to a state before the retraction step is performed.

### Step S30: Stiffening Step

Next, the overtube 10 is stiffened. Specifically, the pump 26 is operated to discharge the air in the tube inner space S through the supply and discharge port 22. Accordingly, in the first example described above, in a case where the air in the tube inner space S is discharged through the supply and discharge port 22, the first region T1 is in a stiffened state, and the second region T2 located on the distal end side with respect to the first region T1 is in a state softer than the stiffened state of the first region T1. Accordingly, it is possible to hold the endoscope insertion part 102 in a stable state without wobbling inside the body by the first region T1, and it is possible to guide the endoscope insertion part 102 in a desired direction by the second region T2. As a result, the insertability of the endoscope insertion part 102 is improved.

### Step S32: Insertion Step

Next, as shown in VIIID of Fig. 8, the endoscope insertion part 102 is pushed into the overtube 10 to insert the endoscope insertion part 102 into the deep portion inside the body.

Thereafter, the bendable part 106 of the endoscope insertion part 102 is caused to protrude forward from the distal end opening of the overtube 10, and a treatment tool (not shown) is caused to be led forward from the treatment tool outlet port of the distal end part 104 to start a treatment such as endoscopic submucosal dissection (ESD). In this case, since the insertability of the endoscope insertion part 102 is improved by the overtube 10 in a stiffened state, the distal end part 104 can be located at an accurate treatment position, and as a result, it is possible to perform an accurate treatment on a treatment target site (lesion portion or the like).

As described above, according to the first embodiment, the overtube 10 comprises the first region T1 that is switchable between a softened state (first state) in which air is supplied to the tube inner space S between the outer tube 30 and the inner tube 32 via the supply and discharge port 22 and a stiffened state (second state) which is a state stiffer than the softened state and in which the air is discharged from the tube inner space S, and the second region T2 that is located on the distal end side with respect to the first region T1 and is configured to be softer than the stiffened state of the first region T1. Accordingly, in a case where the first region T1 is in a stiffened state, it is possible to hold the endoscope insertion part 102 in a stable state inside the body by the first region T1, and it is possible to obtain a sufficient amount of curvature without significantly hindering the bending operation of the endoscope insertion part 102 by the second region T2.

In addition, according to the first embodiment, in the above-described configuration, furthermore, the softened state of the second region T2 is configured to be softer than the softened state of the first region T1. Accordingly, even in a case where an amount of curvature of the endoscope insertion part 102 led out from the distal end of the overtube 10 in a softened state is large, the overtube 10 can be smoothly pushed along the wall surface of the digestive tract without being affected by wrinkles or the like generated in the bendable part 106 of the endoscope insertion part 102.

Therefore, according to the first embodiment, the insertability of the distal end side of the overtube 10 into the digestive tract (particularly, the upper digestive tract) is improved. As a result, it is possible to easily perform an operation of moving the endoscope insertion part 102 and the overtube 10 inside the body, and it is possible to easily and stably guide the endoscope insertion part 102 in a desired direction by using the overtube 10. In particular, the above-described effects are remarkable in a case where the above-described pylorus passage technique is performed.

### Second Embodiment

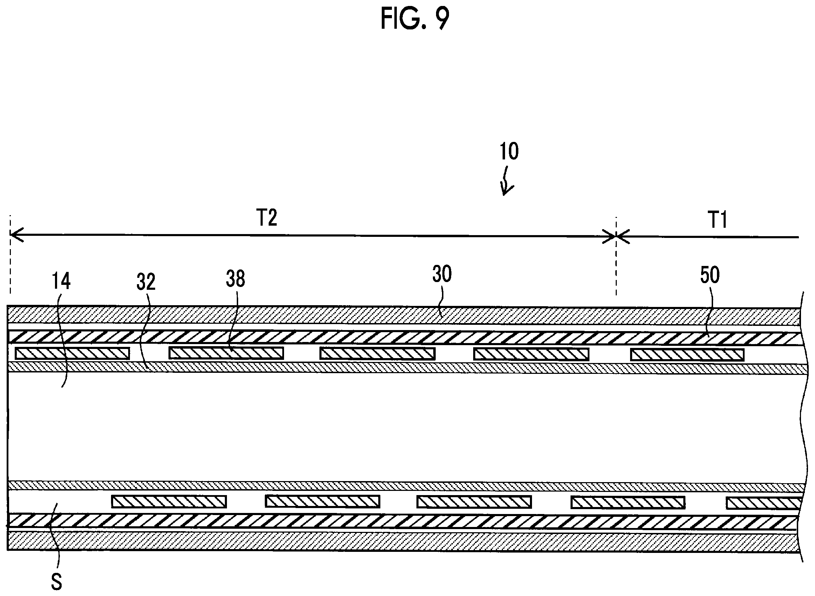

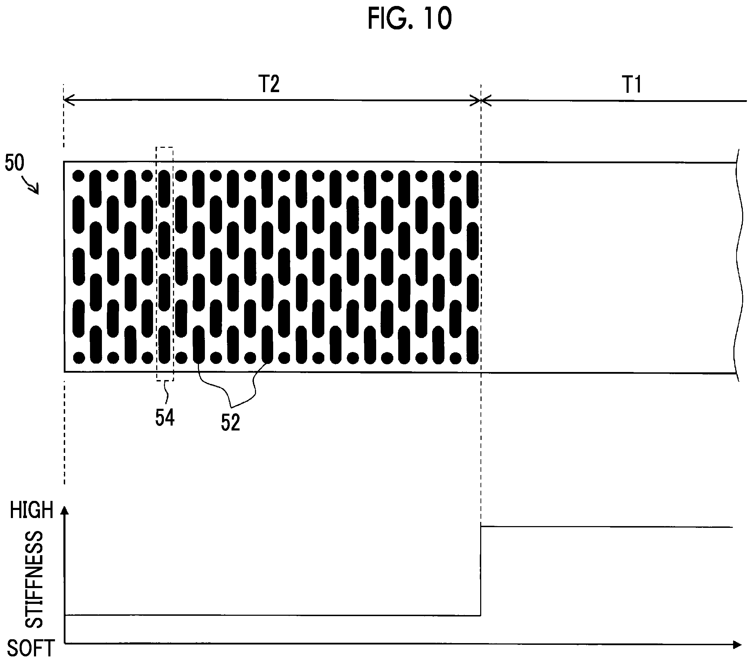

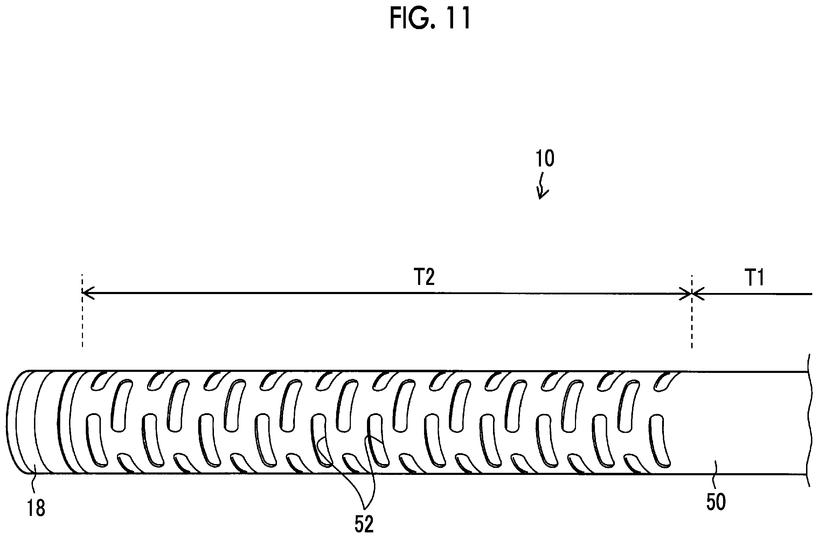

Fig. 9 is a schematic cross-sectional view showing a configuration of the overtube 10 of the second embodiment. Fig. 10 is a view in which a sheet material 50 provided in the overtube 10 of the second embodiment is developed in a planar shape. The lower part of Fig. 10 shows a graph of the stiffness of the overtube 10 in the second embodiment. Fig. 11 is an external view of the overtube 10 of the second embodiment in which the outer tube 30 is omitted.

The overtube 10 of the second embodiment is the same as that of the first embodiment except that a disposition and a shape of the sheet material 50 are different. Hereinafter, a configuration different from that of the first embodiment will be described.

As shown in Fig. 9, in the second embodiment, the sheet material 50 is disposed in both the first region T1 and the second region T2 of the overtube 10. The sheet material 50 has different shapes between the first region T1 and the second region T2 in order to form the second region T2 as a region softer than the first region T1.

Specifically, as shown in Figs. 10 and 11, a region corresponding to the first region T1 in the sheet material 50 is formed in a sheet shape by a smooth surface on which a cut portion 52 is not provided, and a plurality of cut portions 52 are provided in a region corresponding to the second region T2. In the present example, among the plurality of cut portions 52 provided in a region corresponding to the second region T2, most of the cut portions 52 are formed of cut holes elongated in a circumferential direction (vertical direction in Fig. 10), and the remaining cut portions 52 are formed of circular cut holes.

All or some of the cut portions 52 may be formed of cut holes elongated in a direction (lateral direction in Fig. 10) along the longitudinal axis C, or may be formed of circular or elliptical cut holes. In addition, each of the cut portions 52 may be formed of a cut hole having another shape. The cut portions 52 may have holes of a plurality of shapes intermingled as in the example shown in Fig. 10, or may be formed of only holes of a single shape.

As shown in Fig. 10, in a plan view in a case where the sheet material 50 is developed in a planar shape, the plurality of cut portions 52 are disposed according to a regular pattern. Specifically, a plurality of cut portion rows 54 each consisting of a plurality of cut portions 52 arranged at equal intervals in the circumferential direction are arranged along a longitudinal direction (direction along the longitudinal axis C) of the sheet material 50, and the cut portion rows 54 arranged in the longitudinal direction of the sheet material 50 are disposed in a staggered manner to alternate with each other in a state of being offset from each other in the circumferential direction.

In a case where the second region T2 of the overtube 10 can be configured as a region softer than the first region T1, the shape and the arrangement of the cut portions 52 provided in a region of the sheet material 50 corresponding to the first region T1 are not limited to the present example. In addition, at least some, most, substantially all, or all of the plurality of cut portions 52 are not limited to the regular pattern as in the present example, and may be disposed in a random pattern or an irregular pattern. It should be noted that the "regular pattern" means that each of a pitch in the circumferential direction and a pitch in the longitudinal direction (direction along the longitudinal axis C) of the cut portions 52 is regular, and includes a case where each pitch is at equal intervals and a case where each pitch is periodically changed even in a case where each pitch is changed. Therefore, even in a case where shapes or sizes of some of the cut portions 52 are different, the cut portions 52 are included in the regular pattern in a case where each pitch is regular.

According to the second embodiment, since the plurality of cut portions 52 are provided in the region of the sheet material 50 corresponding to the second region T2, the second region T2 of the overtube 12 is configured as a region softer than the first region T1 as shown in a graph in a lower part of Fig. 10. Accordingly, it is possible to easily perform the operation of moving the overtube 10 and the endoscope insertion part 102 inside the body as in the first embodiment. As a result, it is possible to easily and stably guide the endoscope insertion part 102 in a desired direction by using the overtube 10.

In the second embodiment, since the cut portion rows 54 described above are disposed at equal intervals along the longitudinal direction (direction along the longitudinal axis C) of the sheet material 50, a region of the sheet material 50 corresponding to the first region T1 has a constant flexibility along the longitudinal direction of the sheet material 50. In the present specification, the term "constant" does not mean completely constant, but means substantially constant (for example, a variation within a range of plus or minus 10% is allowed).

### Modification Examples of Second Embodiment

Hereinafter, modification examples of the second embodiment will be described.

### First Modification Example

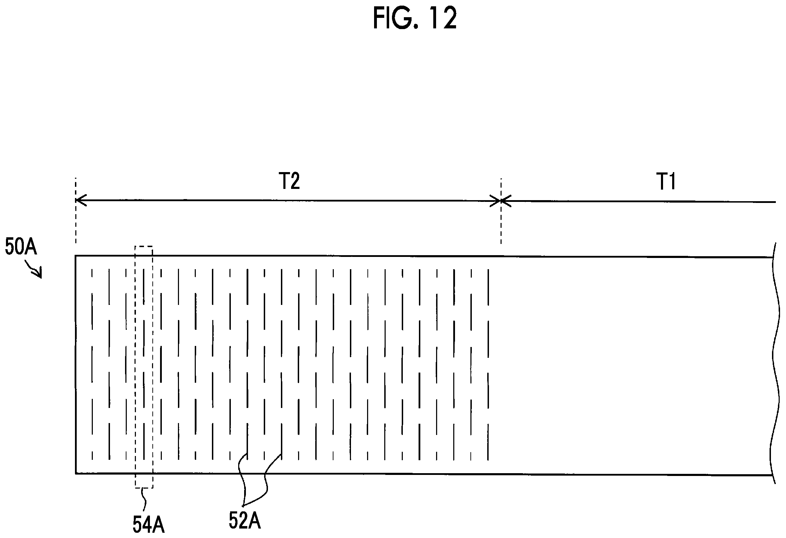

Fig. 12 is a view in which a sheet material 50A according to a first modification example is developed in a planar shape. As shown in Fig. 12, in the first modification example, each of cut portions 52A of the sheet material 50A provided in the second region T2 of the overtube 10 is formed of a cut line provided in the circumferential direction. The cut portions 52A are disposed according to the same regular pattern as in the second embodiment. That is, a plurality of cut portion rows 54A each consisting of a plurality of cut portions 52A arranged at equal intervals in the circumferential direction are arranged along a longitudinal direction (direction along the longitudinal axis C) of the sheet material 50A, and the cut portion rows 54A arranged in the longitudinal direction of the sheet material 50A are disposed in a staggered manner to alternate with each other in a state of being offset from each other in the circumferential direction.

With the cut portions 52A of the first modification example, the second region T2 of the overtube 10 can be configured as a region softer than the first region T1, and the same effect as that of the second embodiment can be obtained.

### Second Modification Example

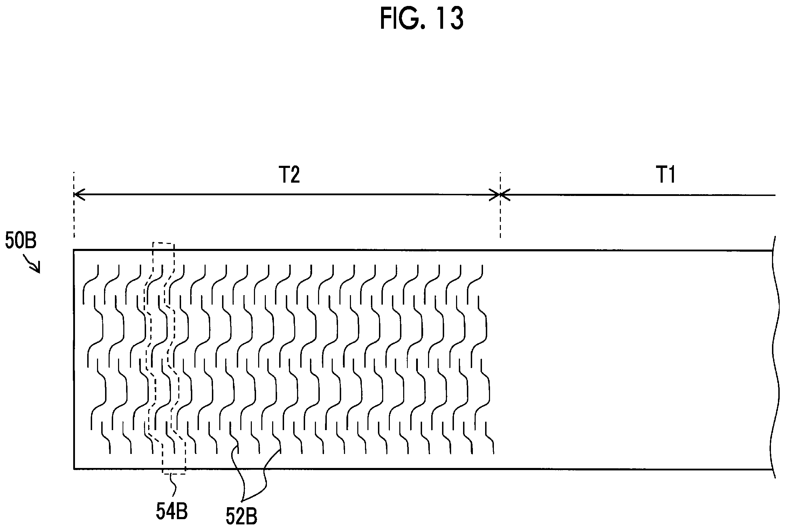

Fig. 13 is a view in which a sheet material 50B according to a second modification example is developed in a planar shape. As shown in Fig. 13, in the second modification example, each of cut portions 52B of the sheet material 50B provided in the second region T2 of the overtube 10 is formed of a cut line having a shape different from that of the first modification example. The cut line constituting the cut portion 52B of the second modification example is formed by combining straight lines or curves in a plurality of directions. For example, the cut portion 52B provided in a central portion of the sheet material 50B in the circumferential direction (vertical direction in Fig. 13) has a central portion in the circumferential direction which is bent toward the proximal end side (right side in Fig. 13) and both end portions which are formed in a straight line shape along the circumferential direction. Further, a plurality of cut portion rows 54B each consisting of a plurality of cut portions 52B arranged at equal intervals in the circumferential direction are arranged at equal intervals along the longitudinal direction (direction along the longitudinal axis C) of the sheet material 50B.

With the cut portions 52B of the second modification example, the second region T2 of the overtube 10 can be configured as a region softer than the first region T1, and the same effect as that of the second embodiment can be obtained.

### Third Modification Example

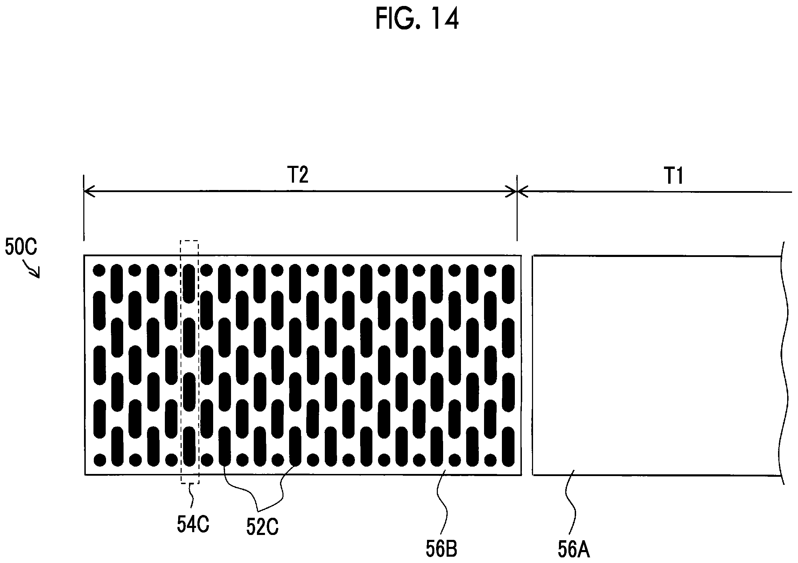

Fig. 14 is a view in which a sheet material 50C according to a third modification example is developed in a planar shape. As shown in Fig. 14, in the third modification example, the sheet material 50C is configured to include a first divided sheet material 56A disposed in the first region T1 and a second divided sheet material 56B disposed in the second region T2 by being divided as separate sheet materials. In this way, the sheet material 50C may be configured by a plurality of divided sheet materials divided for each region.

The cut portion 52C and the cut portion row 54C of the third modified example are the same as the cut portion 52 and the cut portion row 54 of the second embodiment. The sheet materials of other modification examples may also be configured with a plurality of divided sheet materials as in the third modification example.

### Fourth Modified Example

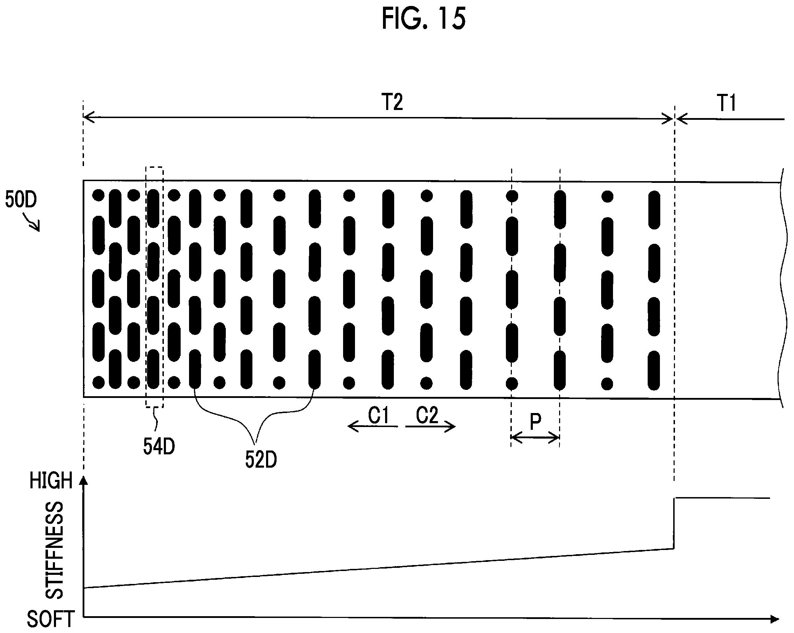

Fig. 15 is a view in which a sheet material 50D according to a fourth modification example is developed in a planar shape. In addition, the lower part of Fig. 15 shows a graph of the stiffness of the overtube 10 in the fourth modification example.

In the fourth modification example shown in Fig. 15, a plurality of cut portions 52D provided in the second region T2 of the sheet material 50D have the same shape as the cut portions 52 of the second embodiment.

In the fourth modification example, a plurality of cut portion rows 54D each consisting of a plurality of cut portions 52D arranged at equal intervals in the circumferential direction are arranged in the longitudinal direction (direction along the longitudinal axis C) of the sheet material 50D, and a pitch P, which is a distance between the cut portion rows 54D in the longitudinal direction of the sheet material 50D, gradually decreases toward the distal end direction C1. Therefore, a region of the sheet material 50D corresponding to the second region T2 is gradually softer toward the distal end direction C1. Accordingly, as shown in a graph in a lower part of Fig. 15, the second region T2 of the overtube 10 is configured as a region (gradation change region) that is softer than the first region T1 and that is gradually softer toward the distal end direction C1.

In addition, a length of the second region T2 of the overtube 10 in the longitudinal direction (direction along the longitudinal axis C) is preferably 5 cm or more and 50 cm or less. In addition, the proximal end of the second region T2 is preferably provided at a position within 50 cm from the distal end of the overtube 10. It is preferable that the same length and position as the fourth modification example are provided also in fifth and sixth modification examples described below.

According to the fourth modification example, the second region T2 of the overtube 10 is gradually softer toward the distal end direction C1, and the distal end side of the second region T2 is configured to be softer than the proximal end side of the second region T2. Accordingly, in a case where the overtube 10 is in a softened state, the overtube 10 can be easily and smoothly pushed into the endoscope insertion part 102 as compared to a case where the second region T2 has a constant flexibility in the longitudinal direction.

It should be noted that, for the cut portion 52D of the fourth modification example, as an example, a case where the cut portion 52A of the second embodiment is applied has been illustrated. However, the present disclosure is not limited thereto, and other modification examples may be applied.

### Fifth Modified Example

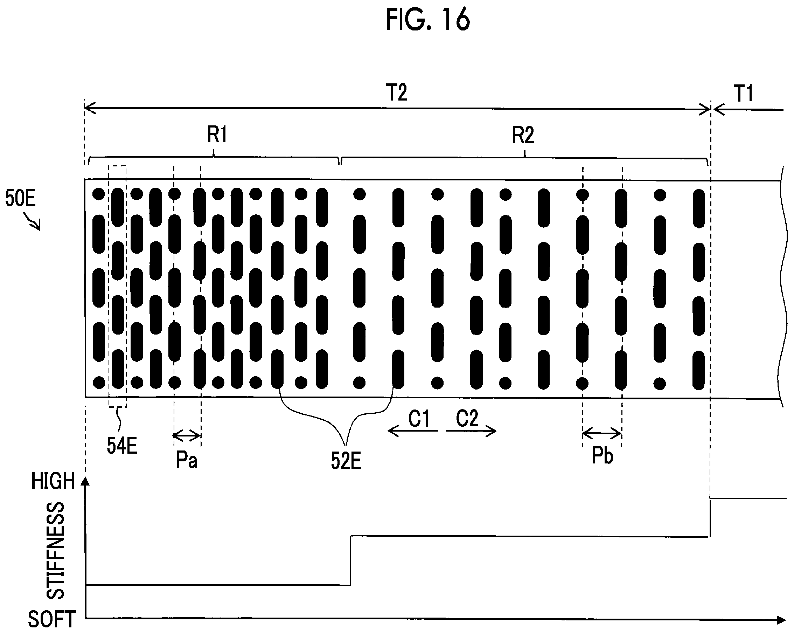

Fig. 16 is a view in which a sheet material 50E according to a fifth modification example is developed in a planar shape. In addition, the lower part of Fig. 16 shows a graph of the stiffness of the overtube 10 in the fifth modification example.

In the fifth modification example shown in Fig. 16, a plurality of cut portions 52E provided in the second region T2 of the sheet material 50E have the same shape as the cut portions 52 of the second embodiment.

In the fifth modification example, a region corresponding to the second region T2 in the sheet material 50E is divided into a plurality of (two in the present example) regions having different stiffnesses from each other. Specifically, the region corresponding to the second region T2 of the sheet material 50E has a distal end-side region R1 (corresponding to a "first interlayer" of the present invention) provided on the distal end side and a proximal end-side region R2 (corresponding to a "second interlayer" of the present invention) provided on the proximal end side.

A plurality of cut portions 52E are provided in each of the distal end-side region R1 and the proximal end-side region R2. In addition, in each of the regions, a pitch of cut portion rows 54E in the longitudinal direction (direction along the longitudinal axis C) is constant. Meanwhile, a pitch Pa of the cut portion rows 54E in the distal end-side region R1 is smaller than a pitch Pb of the cut portion rows 54E in the proximal end-side region R2. Therefore, in a region of the sheet material 50E corresponding to the second region T2, the distal end-side region R1 is softer than the proximal end-side region R2. Accordingly, as shown in a graph in a lower part of Fig. 16, the second region T2 of the overtube 10 is configured as a region that is softer than the first region T1 and that is softer on the distal end side than on the proximal end side.

According to the fifth modification example, the distal end side of the second region T2 is configured to be softer than the proximal end side thereof. Accordingly, similarly to the fourth modification example, in a case where the overtube 10 is in a softened state, the overtube 10 can be easily and smoothly pushed into the endoscope insertion part 102 as compared to a case where the second region T2 has a constant stiffness in the longitudinal direction.

In the fifth modification example, the distal end-side region R1 and the proximal end-side region R2 in the second region T2 may be configured with sheet materials (divided sheet materials) having different stiffnesses from each other. The same applies to a sixth modification example described later.

In addition, in the fifth modification example, an aspect has been described in which the stiffness of the second region T2 in the overtube 10 is changed in two stages. However, the present disclosure is not limited to this, and the stiffness of the second region T2 may be changed in multiple stages as in the sixth modification example described later.

### Sixth Modification Example

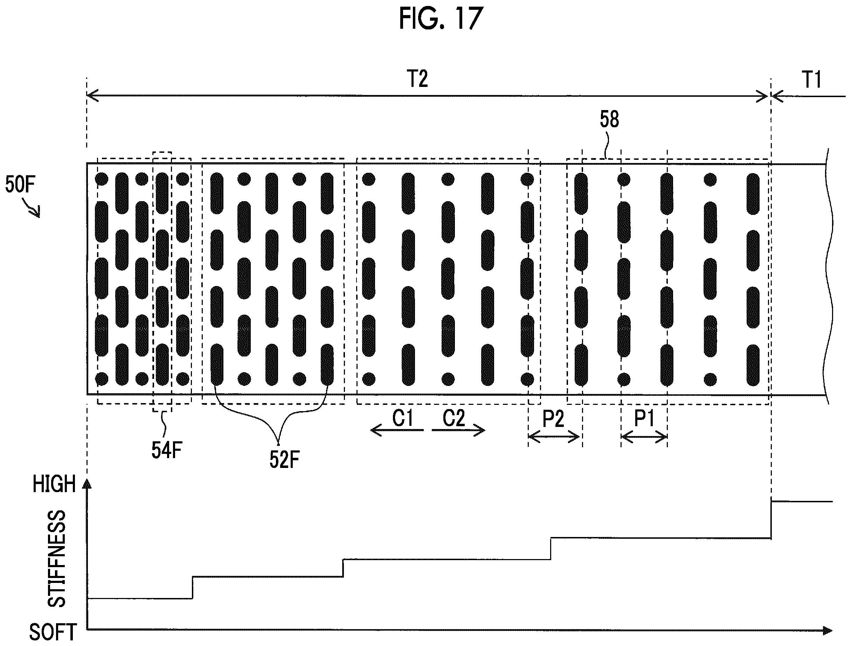

Fig. 17 is a view in which a sheet material 50F according to the sixth modification example is developed in a planar shape. In addition, the lower part of Fig. 17 shows a graph of the stiffness of the overtube 10 in the sixth modification example.

In the sixth modification example shown in Fig. 17, a plurality of cut portions 52F provided in the second region T2 of the sheet material 50F have the same shape as the cut portions 52 of the second embodiment.

In the sixth modification example, a plurality of (five in the present example) cut portion rows 54F form a cut portion group 58. In each of the cut portion groups 58, a pitch P1 of the cut portion rows 54F is constant. Meanwhile, a pitch P2 between the cut portion groups 58 gradually decreases toward the distal end direction C1. Therefore, a region of the sheet material 50F corresponding to the second region T2 is softer in a stepwise manner toward the distal end direction C1. Accordingly, as shown in a graph in a lower part of Fig. 17, the second region T2 of the overtube 10 is configured as a region (stepwise change region) that is softer than the first region T1 and is softer in a stepwise manner toward the distal end direction C1.

According to the sixth modification example, the second region T2 of the overtube 10 is softer in a stepwise manner toward the distal end direction C1, and the distal end side of the second region T2 is configured to be softer than the proximal end side thereof. Accordingly, similarly to the fourth modification example, in a case where the overtube 10 is in a softened state, the overtube 10 can be easily and smoothly pushed into the endoscope insertion part 102 as compared to a case where the second region T2 has a constant stiffness in the longitudinal direction.

### Evaluation of Curvature of Overtube

Next, a result of evaluation of a degree of curvature of the overtube will be described. In this evaluation, a difference in an amount of curvature of the overtube in a case where the endoscope insertion part is operated to be bent is evaluated in a state in which the endoscope insertion part is inserted into the overtube and the distal end of the endoscope insertion part and the distal end of the overtube are substantially matched (a state in which the bendable part of the endoscope insertion part is overlapped with the overtube).

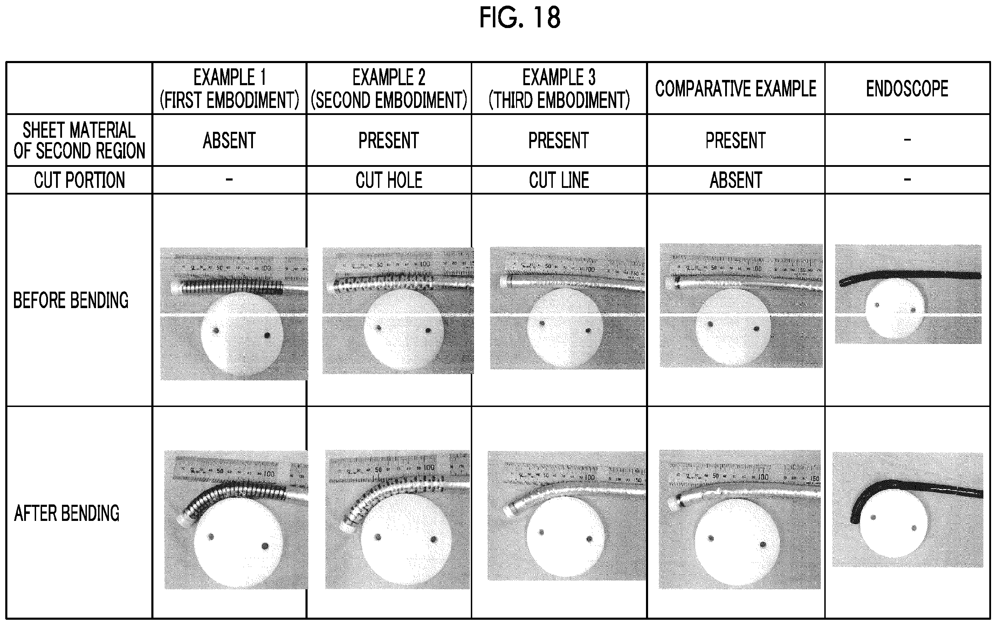

Fig. 18 is a view showing an evaluation result of a degree of curvature of the overtube. In Fig. 18, "Example 1", "Example 2", and "Example 3" in first to third columns excluding an item column are results of evaluations using the overtubes of the first embodiment, the second embodiment, and the first modification example, respectively. A "Comparative Example" in a fourth column corresponds to the above-described related art, in which one sheet material having a smooth surface without a cut portion is disposed over the entire tube body constituting the overtube in the longitudinal direction (that is, the sheet material is disposed in the first region T1 and the second region T2 to make the stiffnesses of the first region T1 and the second region T2 equal to each other). In addition, "Endoscope" in a fifth column shows for reference the results in a case where the endoscope insertion part used in each evaluation is curved alone. The overtube used in the present evaluation is an overtube in a state in which the outer tube is removed, for convenience for evaluation.

As shown in Fig. 18, in the "Comparative Example", an amount of curvature of the overtube is smaller than an amount of curvature in a case where the endoscope insertion part is curved alone. This is because, even in a case where the endoscope insertion part is bent, the endoscope insertion part is restrained by the sheet material disposed in the region corresponding to the second region T2, and the overtube is not sufficiently bent.

On the other hand, in "Example 1", an amount of curvature of the overtube is larger than that of "Comparative Example", and it is possible to obtain substantially the same amount of curvature as in a case where the endoscope insertion part is bent alone. Therefore, the effectiveness of the configuration in which the sheet material is not provided in the region corresponding to the second region T2 was confirmed.

Further, also in the overtube of "Example 2", it is possible to obtain substantially the same amount of curvature as in "Example 1". Therefore, the effectiveness of the configuration in which a plurality of cut portions are provided in the region corresponding to the second region T2 of the sheet material was confirmed.

In addition, the overtube of "Example 3" is configured such that a cut portion provided in the sheet material is formed in a shape (cut line) different from that of "Example 2". In "Example 3", although an amount of curvature of the overtube is smaller than that of "Example 1" and "Example 2", the amount of curvature is larger than that of "Comparative Example", and it was confirmed that a certain effect can be obtained also in "Example 3".

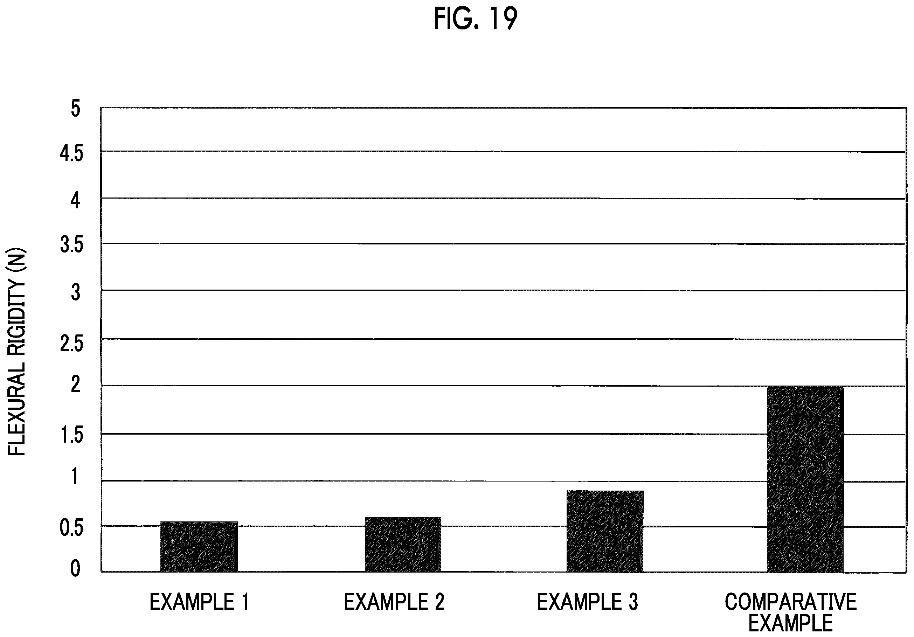

Fig. 19 is a graph showing a result of measuring the stiffness of the second region T2 of the overtube. This measurement method is a method of measuring a stiffness (flexural rigidity) of the overtube in a case where the distal end of the overtube is pushed in a direction orthogonal to the longitudinal direction (direction along the longitudinal axis C) by 25 mm with a position of 70 mm from the distal end of the overtube as a fulcrum, using a force gauge. In Fig. 19, "Example 1", "Example 2", "Example 3", and "Comparative Example" are obtained using the same overtubes as the evaluation results shown in Fig. 18.

According to the evaluation results shown in Fig. 19, in the "Comparative Example", the flexural rigidity of the overtube was 2 N (newton, the same applies hereinafter), whereas the flexural rigidity of the overtube in "Example 1" was 0.56 N and the flexural rigidity of the overtube in "Example 2" was 0.61 N. In addition, in "Example 3", the flexural rigidity was 0.9 N.

As described above, in "Example 1" and "Example 2", the flexural rigidity of the second region T2 is lower than that of "Comparative Example" by about 1/4, and it was confirmed that a sufficient effect can be obtained. In addition, also in "Example 3", the flexural rigidity of the second region T2 is lower than that of "Comparative Example" by about 1/2, and it was confirmed that a certain effect can be obtained.

### Effects

As described above, according to each of the embodiments (including the modification examples, the same applies to hereinafter), the stiffness of the overtube 10 is variable between the stiffened state and the softened state, and the second region T2 of the overtube 10 is configured as a region softer than the first region T1 in any state. As a result, the insertability of the distal end side of the overtube 10 into the digestive tract (particularly, the upper digestive tract) is improved. As a result, it is possible to easily perform an operation of moving the overtube 10 and the endoscope insertion part 102 inside the body, and it is possible to easily and stably guide the endoscope insertion part 102 in a desired direction by using the overtube 10.

### Others

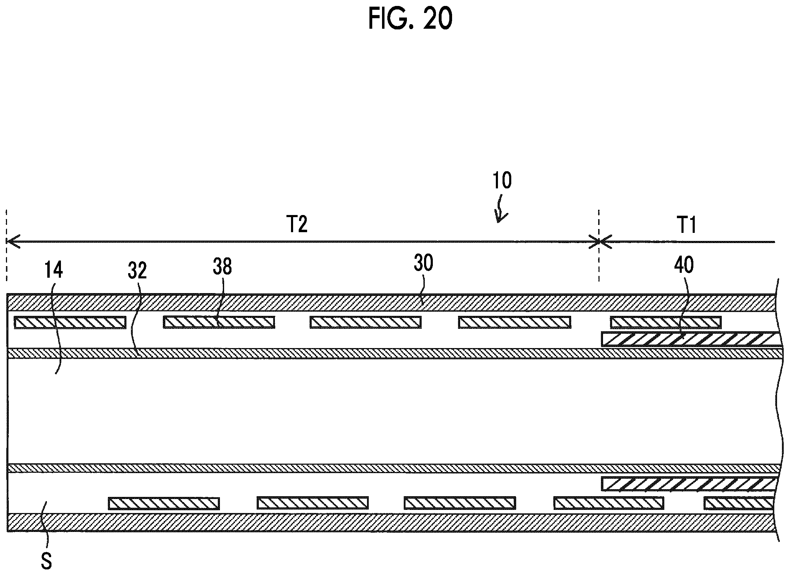

In each of the above-described embodiments, as one preferable aspect, a configuration has been described in which the sheet material 40 or 50 (including 50A to 50F, the same applies hereinafter) is interposed between the outer tube 30 and the shape-deformable body 38. However, the present disclosure is not limited to this, and it is sufficient that the sheet material 40 or 50 is disposed at a position where the sheet material can come into contact with the shape-deformable body 38 between the outer tube 30 and the inner tube 32. For example, as in another configuration example of the overtube 10 of the first embodiment shown in Fig. 20, a configuration in which the sheet material 40 is interposed between the inner tube 32 and the shape-deformable body 38 may be adopted. In this case, at least a part of at least one of the contact surfaces facing each other of the inner tube 32 and the shape-deformable body 38 may be configured as a high friction surface.

In addition, in each of the above-described embodiments, an aspect in which the interlayer provided between the outer tube 30 and the inner tube 32 is formed of the sheet material 40 or 50 has been described. However, the present disclosure is not limited to this, and the interlayer may be formed of a wire-like member or the like. In addition, the sheet material 40 or 50 is not limited to a sheet material in which one sheet is formed in a cylindrical shape. For example, the sheet material 40 or 50 may be formed of a tubular body having a C-shape in cross section orthogonal to the longitudinal axis C, or may have a configuration in which a plurality of strip sheets elongated in a direction along the longitudinal axis C are arranged in the circumferential direction.

In addition, in each of the above-described embodiments, the second region T2 is configured as a region softer than the first region T1 by making the configuration (arrangement or shape) of the sheet material 40 or 50 provided in the overtube 10 different between the first region T1 and the second region T2. In addition to this, or instead of this, a winding pitch (spiral pitch) of the strip-shaped member of the spiral tube constituting the shape-deformable body 38 may be made different between the first region T1 and the second region T2. Even in this case, the second region T2 of the overtube 10 can be configured as a region softer than the first region T1.

In addition, in each of the above-described embodiments, an aspect in which the shape-deformable body 38 is formed of the spiral pipe has been described as a preferable aspect. However, the present disclosure is not limited to this, and a known configuration may be applied as long as the shape-deformable body 38 can be deformed following the shape of the overtube 10. For example, an articulated ring member disclosed in WO2022/181200A described above can also be applied as the shape-deformable body 38. Since the articulated ring member is well known, a detailed description thereof will be omitted here.

Further, in each of the above-described embodiments, an example in which the endoscope insertion part 102 is applied as a medical instrument guided to the overtube according to the embodiment of the present invention has been described. However, the present disclosure is not limited to this, and can also be applied to, for example, a medical treatment tool such as a manipulator.

### Explanation of References

1: endoscope apparatus
10: overtube
10a: distal end
10b: proximal end
14: insertion path
16: tube outer peripheral wall
18: distal end cap
20: proximal end cap
22: supply and discharge port
24: fluid supply and discharge tube
26: pump
30: outer tube
32: inner tube
38: shape-deformable body
40: sheet material
50: sheet material
50A: sheet material
50B: sheet material
50C: sheet material
50D: sheet material
50E: sheet material
50F: sheet material
52: cut portion
52A: cut portion
52B: cut portion
52C: cut portion
52D: cut portion
52E: cut portion
52F: cut portion
54A: cut portion row
54B: cut portion row
54C: cut portion row
54D: cut portion row
54E: cut portion row
54F: cut portion row
56A: first divided sheet material
56B: second divided sheet material
58: cut portion group
100: endoscope
102: endoscope insertion part
104: distal end part
104a: distal end surface
106: bendable part
108: soft part
110: hand operating part
200: stomach
202: pylorus
204: small bulge portion
C: longitudinal axis
C1: distal end direction
C2: proximal end direction
T1: first region
T2: second region
P: pitch
Pa: pitch
Pb: pitch
P1: pitch
P2: pitch
R1: distal end-side region
R2: proximal end-side region
S: space (tube inner space)

## Claims

1. An overtube having an insertion path into which a medical instrument is inserted, the overtube comprising:
a supply and discharge port that is located on a proximal end side and through which a fluid is supplied and discharged;
a first region that is located on a distal end side with respect to the supply and discharge port and includes an outer tube having a flexibility, an inner tube having a flexibility, and a shape-deformable body that is deformable and is provided between the outer tube and the inner tube; and
a second region that is located on the distal end side with respect to the first region and includes the outer tube and the inner tube,
wherein the first region is switchable between a first state in which the fluid is supplied to a space between the outer tube and the inner tube via the supply and discharge port and a second state that is stiffer than the first state and in which the fluid is discharged, and
the second region is softer than the second state of the first region.

2. The overtube according to claim 1,
wherein the second region is softer than the first state of the first region.

3. The overtube according to claim 1 or 2,
wherein a distal end side of the second region is softer than a proximal end side of the second region.

4. The overtube according to any one of claims 1 to 3,
wherein the second region includes the shape-deformable body, and
the second region is switchable between the first state in which the fluid is supplied to the space between the outer tube and the inner tube via the supply and discharge port and the second state in which the fluid is discharged.

5. The overtube according to any one of claims 1 to 4,
wherein the first state of the second region is softer than the first state of the first region, and
the second state of the second region is softer than the second state of the first region.

6. The overtube according to any one of claims 1 to 5,
wherein the first region has an interlayer that is provided between the outer tube and the inner tube and is in contact with the shape-deformable body, and
a first contact surface provided in the shape-deformable body and a second contact surface provided in the interlayer and facing the first contact surface come into contact with each other in the second state, so that the first region becomes stiff.

7. The overtube according to any one of claims 1 to 6,
wherein the second region includes the interlayer different in configuration from the interlayer of the first region.

8. The overtube according to claim 7,
wherein the interlayer of the second region has a constant flexibility along a longitudinal direction.

9. The overtube according to claim 7,
wherein the interlayer of the second region is softer on a distal end side than on a proximal end side.

10. The overtube according to claim 9,
wherein the interlayer of the second region includes a first interlayer provided on the distal end side and a second interlayer provided on the proximal end side, and the first interlayer is softer than the second interlayer,
or
wherein the interlayer of the second region is gradually softer toward the distal end side.

11. The overtube according to any one of claims 7 to 10,
wherein the interlayer is formed of a sheet material provided from the first region to the second region, and
a cut portion is provided in the sheet material of the second region, and
preferably, the sheet material of the first region is formed of a smooth surface.

12. The overtube according to any one of claims 6 to 11,
wherein at least a part of any one of the first contact surface or the second contact surface includes a high friction surface.

13. The overtube according to any one of claims 6 to 12,
wherein the interlayer is provided between the outer tube and the shape-deformable body.

14. The overtube according to any one of claims 1 to 13,
wherein the shape-deformable body includes a spiral tube formed by spirally winding a strip-shaped member on an outer peripheral side of the inner tube.

15. The overtube according to any one of claims 1 to 14,
wherein a length of the second region in a longitudinal direction is 5 cm or more and 20 cm or less, and a proximal end of the second region is provided at a position within 20 cm from a distal end,
and/or
wherein a length of the first region in a longitudinal direction is longer than a length of the second region.

## Patentansprüche

1. Überrohr, das einen Einführweg aufweist, in den ein medizinisches Instrument eingeführt wird, wobei das Überrohr umfasst:
eine Versorgungs- und Abgabeöffnung, die sich auf einer proximalen Endseite befindet und durch die ein Fluid zugeführt und abgegeben wird;
einen ersten Bereich, der sich in Bezug auf die Versorgungs- und Abgabeöffnung auf einer distalen Endseite befindet und ein Außenrohr, das eine Flexibilität aufweist, ein Innenrohr, das eine Flexibilität aufweist, und einen formverformbaren Körper, der verformbar ist und zwischen dem Außenrohr und dem Innenrohr vorgesehen ist, enthält; und
einen zweiten Bereich, der sich in Bezug auf den ersten Bereich auf der distalen Endseite befindet und das Außenrohr und das Innenrohr enthält,
wobei der erste Bereich zwischen einem ersten Zustand, in dem das Fluid via die Versorgungs- und Abgabeöffnung einem Raum zwischen dem Außenrohr und dem Innenrohr zugeführt wird, und einem zweiten Zustand, der steifer als der erste Zustand ist und in dem das Fluid abgegeben wird, umschaltbar ist, und
der zweite Bereich weicher als der zweite Zustand des ersten Bereichs ist.

2. Überrohr nach Anspruch 1,
wobei der zweite Bereich weicher als der erste Zustand des ersten Bereichs ist.

3. Überrohr nach Anspruch 1 oder 2,
wobei eine distale Endseite des zweiten Bereichs weicher als eine proximale Endseite des zweiten Bereichs ist.

4. Überrohr nach einem der Ansprüche 1 bis 3,
wobei der zweite Bereich den formverformbaren Körper enthält, und
der zweite Bereich zwischen dem ersten Zustand, in dem das Fluid via die Versorgungs- und Abgabeöffnung dem Raum zwischen dem Außenrohr und dem Innenrohr zugeführt wird, und dem zweiten Zustand, in dem das Fluid abgegeben wird, umschaltbar ist.

5. Überrohr nach einem der Ansprüche 1 bis 4,
wobei der erste Zustand des zweiten Bereichs weicher als der erste Zustand des ersten Bereichs ist, und
der zweite Zustand des zweiten Bereichs weicher als der zweite Zustand des ersten Bereichs ist.

6. Überrohr nach einem der Ansprüche 1 bis 5,
wobei der erste Bereich eine Zwischenschicht aufweist, die zwischen dem Außenrohr und dem Innenrohr vorgesehen ist und mit dem formverformbaren Körper in Kontakt steht, und
eine erste Kontaktfläche, die bei dem formverformbaren Körper vorgesehen ist, und eine zweite Kontaktfläche, die bei der Zwischenschicht vorgesehen ist und der ersten Kontaktfläche zugewandt ist, in dem zweiten Zustand miteinander in Kontakt kommen, so dass der erste Bereich steif wird.

7. Überrohr nach einem der Ansprüche 1 bis 6,
wobei der zweite Bereich die Zwischenschicht enthält, die bezüglich Konfiguration von der Zwischenschicht des ersten Bereichs unterschiedlich ist.

8. Überrohr nach Anspruch 7,
wobei die Zwischenschicht des zweiten Bereichs eine konstante Flexibilität entlang einer Längsrichtung aufweist.

9. Überrohr nach Anspruch 7,
wobei die Zwischenschicht des zweiten Bereichs auf einer distalen Endseite weicher als auf einer proximalen Endseite ist.

10. Überrohr nach Anspruch 9,
wobei die Zwischenschicht des zweiten Bereichs eine erste Zwischenschicht, die auf der distalen Endseite vorgesehen ist, und eine zweite Zwischenschicht, die auf der proximalen Endseite vorgesehen ist, enthält und die erste Zwischenschicht weicher als die zweite Zwischenschicht ist,
oder
wobei die Zwischenschicht des zweiten Bereichs zu der distalen Endseite hin allmählich weicher ist.

11. Überrohr nach einem der Ansprüche 7 bis 10,
wobei die Zwischenschicht aus einem Blattmaterial gebildet ist, das von dem ersten Bereich bis zu dem zweiten Bereich vorgesehen ist, und
ein geschnittener Abschnitt bei dem Blattmaterial des zweiten Bereichs vorgesehen ist, und
bevorzugt das Blattmaterial des ersten Bereichs aus einer glatten Oberfläche gebildet ist.

12. Überrohr nach einem der Ansprüche 6 bis 11,
wobei mindestens ein Teil von der ersten Kontaktfläche oder der zweiten Kontaktfläche eine Oberfläche mit hoher Reibung enthält.

13. Überrohr nach einem der Ansprüche 6 bis 12,
wobei die Zwischenschicht zwischen dem Außenrohr und dem formverformbaren Körper vorgesehen ist.

14. Überrohr nach einem der Ansprüche 1 bis 13,
wobei der formverformbare Körper ein spiralförmiges Rohr enthält, das durch spiralförmiges Wickeln eines streifenförmigen Elements an einer Außenumfangsseite des Innenrohrs gebildet ist.

15. Überrohr nach einem der Ansprüche 1 bis 14,
wobei eine Länge des zweiten Bereichs in einer Längsrichtung 5 cm oder mehr und 20 cm oder weniger beträgt und ein proximales Ende des zweiten Bereichs an einer Position innerhalb von 20 cm von einem distalen Ende vorgesehen ist,
und/oder
wobei eine Länge des ersten Bereichs in einer Längsrichtung länger als eine Länge des zweiten Bereichs ist.

## Revendications

1. Surtube comportant un chemin d'insertion dans lequel est inséré un instrument médical, le surtube comprenant :
un orifice d'alimentation et d'évacuation qui est situé sur un côté d'extrémité proximale et par lequel un fluide est alimenté et évacué ;
une première région qui est située sur un côté d'extrémité distale par rapport à l'orifice d'alimentation et d'évacuation et inclut un tube extérieur ayant une flexibilité, un tube intérieur ayant une flexibilité, et un corps déformable en forme qui est déformable et
est prévu entre le tube extérieur et le tube intérieur ; et
une deuxième région qui est située sur le côté d'extrémité distale par rapport à la première région et inclut le tube extérieur et le tube intérieur,
dans lequel la première région est commutable entre un premier état dans lequel le fluide est alimenté vers un espace entre le tube extérieur et le tube intérieur via l'orifice d'alimentation et d'évacuation, et un deuxième état qui est plus rigide que le premier état et dans lequel le fluide est évacué, et
la deuxième région est plus souple que le deuxième état de la première région.

2. Surtube selon la revendication 1,
dans lequel la deuxième région est plus souple que le premier état de la première région.

3. Surtube selon la revendication 1 ou la revendication 2,
dans lequel un côté d'extrémité distale de la deuxième région est plus souple qu'un côté d'extrémité proximale de la deuxième région.

4. Surtube selon l'une quelconque des revendications 1 à 3,
dans lequel la deuxième région inclut le corps déformable en forme, et
la deuxième région est commutable entre le premier état dans lequel le fluide est alimenté vers l'espace entre le tube extérieur et le tube intérieur via l'orifice d'alimentation et d'évacuation, et le deuxième état dans lequel le fluide est évacué.

5. Surtube selon l'une quelconque des revendications 1 à 4,
dans lequel le premier état de la deuxième région est plus souple que le premier état de la première région, et
le deuxième état de la deuxième région est plus souple que le deuxième état de la première région.

6. Surtube selon l'une quelconque des revendications 1 à 5,
dans lequel la première région comporte une couche intermédiaire qui est prévue entre le tube extérieur et le tube intérieur et est en contact avec le corps déformable en forme, et
une première surface de contact prévue dans le corps déformable en forme et une deuxième surface de contact prévue dans la couche intermédiaire et faisant face à la première surface de contact entrent en contact l'une avec l'autre dans le deuxième état, de sorte que la première région devient rigide.

7. Surtube selon l'une quelconque des revendications 1 à 6,
dans lequel la deuxième région inclut la couche intermédiaire de configuration différente de celle de la couche intermédiaire de la première région.

8. Surtube selon la revendication 7,
dans lequel la couche intermédiaire de la deuxième région a une flexibilité constante le long d'une direction longitudinale.

9. Surtube selon la revendication 7,
dans lequel la couche intermédiaire de la deuxième région est plus souple d'un côté d'extrémité distale que d'un côté d'extrémité proximale.

10. Surtube selon la revendication 9,
dans lequel la couche intermédiaire de la deuxième région inclut une première couche intermédiaire prévue sur le côté d'extrémité distale et une deuxième couche intermédiaire prévue sur le côté d'extrémité proximale, et la première couche intermédiaire est plus souple que la deuxième couche intermédiaire,
ou
dans lequel la couche intermédiaire de la deuxième région est progressivement plus souple vers le côté d'extrémité distale.

11. Surtube selon l'une quelconque des revendications 7 à 10,
dans lequel la couche intermédiaire est formée d'un matériau en feuille prévu de la première région à la deuxième région, et
une partie coupée est prévue dans le matériau en feuille de la deuxième région, et de préférence, le matériau en feuille de la première région est formé d'une surface lisse.

12. Surtube selon l'une quelconque des revendications 6 à 11,
dans lequel au moins une partie de l'une quelconque de la première surface de contact ou de la deuxième surface de contact inclut une surface à friction élevée.

13. Surtube selon l'une quelconque des revendications 6 à 12,
dans lequel la couche intermédiaire est prévue entre le tube extérieur et le corps déformable en forme.

14. Surtube selon l'une quelconque des revendications 1 à 13,
dans lequel le corps déformable en forme inclut un tube spiral formé en enroulant en spirale un élément en forme de bande sur un côté périphérique externe du tube intérieur.

15. Surtube selon l'une quelconque des revendications 1 à 14,
dans lequel une longueur de la deuxième région dans une direction longitudinale est de 5 cm ou plus et de 20 cm ou moins, et une extrémité proximale de la deuxième région est prévue à une position à moins de 20 cm d'une extrémité distale,
et/ou
dans lequel une longueur de la première région dans une direction longitudinale est plus longue qu'une longueur de la deuxième région.
